# EUROPEAN PATENT APPLICATION

(11) **EP 4 748 925 A1**
(43) Date of publication of application: **27.05.2026**
(21) Application number: 25745465.2
(22) Date of filing: 16.01.2025
(51) Int. Cl.: C12N 9/12, C12N 15/77, C12P 13/22, C12P 13/24

(54) **NOVEL RIBOSE PHOSPHATE DIPHOSPHOKINASE VARIANT AND L-AMINO ACID PRODUCTION METHOD USING SAME**

(30) Priority: 26.01.2024 KR 20240012659
(71) Applicant: CJ Cheiljedang Corporation, Seoul 04560 (KR)
(72) Inventor: LEE, Sumin, Seoul 04560 (KR); PARK, Seul-Gi, Seoul 04560 (KR); JUNG, Moo Young, Seoul 04560 (KR); PARK, Soe-hee, Seoul 04560 (KR); KIM, Seon Hye, Seoul 04560 (KR)
(74) Representative: Meissner Bolte Partnerschaft mbB
(86) International application number: PCT/KR2025/000948
(87) International publication number: WO 2025/159448

(57) **Abstract**

Provided are a novel variant polypeptide of ribose phosphate diphosphokinase; a polynucleotide encoding the variant polypeptide; a microorganism comprising the variant polypeptide or the polynucleotide encoding the variant polypeptide; a method of producing an L-amino acid, the method comprising the step of culturing the microorganism in a medium; and use of the polypeptide, polynucleotide, and microorganism in producing an L-amino acid.

## Description

### [Technical Field]

The present disclosure relates to a novel variant polypeptide of ribose phosphate diphosphokinase; a polynucleotide encoding the variant polypeptide; a microorganism comprising the variant polypeptide or the polynucleotide encoding the variant polypeptide; and a method of producing an L-amino acid, the method comprising the step of culturing the microorganism in a medium.

### [Background Art]

A process of producing target materials (e.g., amino acids) in microorganisms has been studied in various ways as an environmentally friendly and safe production method, and among them, research has been continuously conducted to produce large quantities of target materials from microorganisms of the *Corynebacterium* sp. A microorganism of the *Corynebacterium* sp., particularly, *Corynebacterium glutamicum,* is a gram-positive microorganism that is widely used to produce L-amino acids and other useful materials.

L-amino acids are the basic structural units of proteins and are used as important materials for pharmaceutical raw materials, food additives, animal feeds, nutritional supplements, pesticides, bactericides, etc. For the production of L-amino acids and other useful materials, various studies are conducted to develop microorganisms with high-efficiency production and technologies for fermentation processes. For example, target material-specific approaches, such as increasing the expression of genes encoding enzymes involved in L-lysine biosynthesis or removing genes unnecessary for biosynthesis, are mainly used (US 8048650 B2).

L-tryptophan is one of the essential amino acids, and has been widely used in feed additives, pharmaceutical raw materials such as transfusion agents, etc., health food materials, etc. L-tryptophan may be produced by a chemical synthesis method, an enzyme reaction method, a fermentation method, etc., but a direct fermentation method using a microorganism is mainly used at present. According to previous studies, it was demonstrated by intracellular quantitative analysis that the highest level of energy is required for biosynthesis of tryptophan among 20 amino acids (Proc. Natl. Acad. Sci. USA,(2002) V99, pp3695-3700). Therefore, research is needed to effectively increase L-tryptophan production.

L-histidine is one of the 20 standard amino acids and is classified as an essential amino acid for growing children. L-histidine is involved in important physiological processes such as antioxidation and immune regulation, etc., and is used in the medical industry as a raw material for gastric ulcer treatment, circulatory system treatment, and amino acid transfusion. Since histidine is particularly abundant in hemoglobin, it is mainly produced through a protein hydrolysis extraction method using blood meal as a raw material, but this has disadvantages such as low efficiency and environmental pollution, etc. It is possible to produce L-histidine through microbial fermentation, but large-scale industrialization has not yet been achieved. Therefore, research is still needed to effectively increase L-histidine production.

Meanwhile, phosphoribosyl pyrophosphate (PRPP) is one of the precursors required for tryptophan and histidine biosynthesis, and is essential for improving tryptophan or histidine production. A previous study reported that when ribose phosphate diphosphokinase is overexpressed, the supply of phosphoribosyl pyrophosphate increases, leading to an increase in histidine biosynthesis (EP 1529839 A1).

### [Disclosure]

### [Technical Problem]

An object to be solved in the present disclosure is to provide a variant polypeptide of ribose phosphate diphosphokinase, in which an amino acid corresponding to position 239 in an amino acid sequence of SEQ ID NO: 1 is substituted with a different amino acid; a polynucleotide encoding the variant polypeptide; a microorganism comprising the variant polypeptide or the polynucleotide encoding the variant polypeptide; a method of producing an L-amino acid, the method comprising the step of culturing the microorganism in a medium; and use of the polypeptide, polynucleotide, or microorganism in producing an L-amino acid.

### [Technical Solution]

An aspect of the present disclosure provides a variant polypeptide of ribose phosphate diphosphokinase, in which an amino acid corresponding to position 239 in an amino acid sequence of SEQ ID NO: 1 is substituted with a different amino acid.

In one specific embodiment, with regard to the variant polypeptide, the amino acid corresponding to position 239 in the amino acid sequence of SEQ ID NO: 1 may be substituted with valine, proline, asparagine, methionine, glycine, or tyrosine.

In another specific embodiment, the variant polypeptide may consist of any one amino acid sequence selected from SEQ ID NOS: 15 to 20.

Another aspect of the present disclosure provides a polynucleotide encoding the variant polypeptide of ribose phosphate diphosphokinase.

Still another aspect of the present disclosure provides a microorganism comprising the variant polypeptide of ribose phosphate diphosphokinase or the polynucleotide encoding the variant polypeptide.

In one specific embodiment, the microorganism may have an increased L-amino acid production ability, as compared to an unmodified microorganism.

With regard to the microorganism according to any one of the aforementioned specific embodiments, the microorganism may be a microorganism of the *Corynebacterium* sp.

With regard to the microorganism according to any one of the aforementioned specific embodiments, the microorganism of the *Corynebacterium* sp. may be *Corynebacterium glutamicum.*

With regard to the microorganism according to any one of the aforementioned specific embodiments, the L-amino acid may be any one or more selected from the group consisting of L-tryptophan and L-histidine.

Still another aspect of the present disclosure provides a method of producing an L-amino acid, the method comprising the step of culturing the microorganism in a medium.

In one specific embodiment, the method may further comprise the step of recovering a target material from the cultured microorganism, a culture of the microorganism, a fermentation product of the microorganism, or the culture medium.

Still another aspect of the present disclosure provides a composition for producing an L-amino acid, the composition comprising the variant polypeptide of ribose phosphate diphosphokinase; the polynucleotide encoding the variant polypeptide; the microorganism comprising the variant polypeptide or the polynucleotide encoding the variant polypeptide; the culture of the microorganism; a combination of two or more thereof.

Still another aspect of the present disclosure provides use of the variant polypeptide of ribose phosphate diphosphokinase; the polynucleotide encoding the variant polypeptide; the microorganism comprising the variant polypeptide or the polynucleotide encoding the variant polypeptide; the culture of the microorganism in producing an L-amino acid.

### [Advantageous Effects]

When a microorganism comprising a variant polypeptide of ribose phosphate diphosphokinase of the present disclosure is cultured, it is possible to produce an L-amino acid with a high yield, as compared to an unmodified microorganism.

### [Detailed Description of Preferred Embodiments]

The present disclosure will be described in detail as follows. Meanwhile, each description and embodiment disclosed in this disclosure may also be applied to other descriptions and embodiments. That is, all combinations of various elements disclosed in this disclosure fall within the scope of the present disclosure. Further, the scope of the present disclosure is not limited by the specific description described below. Further, a number of papers and patent documents are referenced and cited throughout this specification. The disclosures of the cited papers and patent documents are incorporated herein by reference in their entirety to further clarify the level and scope of the subject matter to which the present disclosure pertains.

### Definition

As used in the specification and appended claims of the present disclosure, the singular forms ("a", "an", and "the") comprise plural referents unless the context states otherwise. Unless the context indicates otherwise, singular terms shall comprise the plural, and plural terms shall comprise the singular. As used in the specification and appended claims of the present disclosure, unless stated otherwise, the use of "or" may be used to comprise "and/or".

As used herein, the term "about" may be presented before a particular numerical value. The term "about" used herein comprises not only the exact number recited after the term, but also a range that is near or close to that number. Considering the context in which the number is presented, it may be determined whether any number is close to or near the particular number presented. For example, the term "about" may refer to a range from -10% to +10% of a numerical value. For another example, the term "about" may refer to a range from -5% to +5% of a given numerical value, but is not limited thereto.

As used herein, the descriptions such as the terms "first, second, third..." "i), ii), iii),," or "(a), (b), (c), (d),," may be used to distinguish respective constitutions. When these terms are used in reference to steps of a method, use, or assay, they do not mean that the constitutions are performed continually or sequentially, for example, there may be no time interval between these steps, or they may be performed concurrently, or may be performed sequentially, reversely, or randomly with intervals of seconds, minutes, hours, days, or months.

As used herein, the term "consisting of" means that the total ratio of specific features, steps, components, or other elements recited after the term is 100%. The features, steps, components, or other elements that are recited after the term "consisting of" may be essential or mandatory. For example, in addition to the features, steps, components, or other elements recited after the term "consisting of", any other features, steps, components, or other elements, or non-essential features, steps, components, or other elements may be excluded.

As used herein, the term "consisting essentially of" may mean that, in the case where the features, steps, components, or other elements of the object claimed herein are not substantially affected by the presence of one or more unspecified features, steps, components, or other elements, the one or more unspecified features, steps, components, or other elements may be present.

As used herein, the term "comprising" means the presence of features, steps, components, or other elements recited after the term, and does not exclude the presence of additional one or more features, steps, components, or other elements. The features, steps, components, or other elements recited after the term "comprising" herein may be essential or mandatory. However, in some embodiments, the term may further comprise any other or non-essential features, steps, components, or other elements.

### Protein, Polypeptide, Variant Polypeptide

As used herein, the term "protein" or "polypeptide" means a polymer or oligomer of consecutive amino acid residues. In the present disclosure, "polypeptide," "protein" and "peptide" may be used interchangeably.

As used herein, the term "mature polypeptide or mature protein" means a polypeptide or protein in a form without a signal sequence or propeptide sequence. A mature polypeptide or mature protein may be a functional form of a polypeptide or protein. The mature polypeptide or mature protein may be a polypeptide in a final form after translation; and/or post-translational modification. For example, examples of the posttranslational modification comprise N-terminal processing, C-terminal truncation, glycosylation, phosphorylation, leader sequence removal etc., but are not limited thereto.

As used herein, the term "wild-type" refers to a naturally-occurring state without artificial modification. When the term "wild-type" is used in reference to a polypeptide, it refers to a naturally occurring polypeptide, which does not have artificial mutations (substitutions, insertions, deletions, etc.) at one or more amino acid positions. Similarly, when the term "wild-type" is used in reference to a polynucleotide, it means a naturally occurring polynucleotide not having artificial modifications (substitutions, insertions, deletions, etc.) at one or more nucleotide positions. However, polynucleotides encoding wild-type polypeptides are not limited to naturally occurring polynucleotides, and comprise sequences encoding any wild-type polypeptides.

As used herein, a parent sequence or backbone refers to a reference sequence in which modification is introduced to create a variant polypeptide. That is, the parent sequence may be served as a starting sequence in which modification such as substitutions, additions, and/or deletions, etc. may be introduced. The parent sequence may be naturally-occurring or wild-type, or a variant in which one or more substitutions, insertions or deletions have occurred in the naturally-occurring or wild-type, or may be an artificially synthesized sequence.

As used herein, the term "reference sequence" means a sequence that serves as a reference for specifying the position N in a specific amino acid sequence. For example, when a specific amino acid sequence and a reference sequence are aligned through a sequence alignment known in the art, and based on this, when each amino acid residue of the specific amino acid sequence is numbered with reference to the numerical position of the amino acid residue of the reference sequence, the position of the amino acid corresponding to the position N of the reference sequence may be determined within the specific amino acid sequence.

As used herein, the term "position N" in an amino acid sequence may mean an amino acid position corresponding to the position N, in addition to the position N. Specifically, the position N may comprise an amino acid position corresponding to an arbitrary amino acid residue in a mature polypeptide, disclosed in a specific amino acid sequence. The specific amino acid sequence may be an amino acid sequence of a reference sequence.

As used herein, the term "corresponding to" refers to an amino acid residue at a position listed in a polypeptide, or an amino acid residue that is similar to, identical to, or homologous to a residue listed in a polypeptide. Identifying the amino acid at the corresponding position may be determining a specific amino acid in a sequence that refers to a specific sequence. As used herein, the term "corresponding region" generally refers to a similar or corresponding position in an amino acid sequence of a related protein or a reference protein.

In the present disclosure, the amino acid sequence of SEQ ID NO: 1 may be used as a reference sequence in determining the position of an amino acid in an arbitrary amino acid sequence.

In other words, the amino acid sequence of SEQ ID NO: 1, as disclosed herein, may be used to determine the corresponding amino acid residues in an arbitrary polypeptide. Unless indicated otherwise in the present disclosure, residues of a particular amino acid sequence are numbered based on the amino acid sequence of SEQ ID NO: 1.

For example, an arbitrary amino acid sequence is aligned with the amino acid sequence of SEQ ID NO: 1, and based on the alignment, each amino acid residue of the arbitrary amino acid sequence may be numbered with reference to the numerical position of the amino acid residue corresponding to the amino acid residue of SEQ ID NO: 1. For example, a sequence alignment algorithm, such as that described herein, may identify the position of an amino acid or a position where modifications such as substitutions, insertions or deletions, etc. occur, compared to a query sequence (also referred to as a "reference sequence").

In such alignment, for example, the Needleman-Wunsch algorithm (Needleman and Wunsch, 1970, J. Mol. Biol. 48: 443-453), the Needle program of the EMBOSS package (EMBOSS: The European Molecular Biology Open Software Suite, Rice et al., 2000), Trends Genet. 16: 276-277), etc. may be used, but is not limited thereto, and a sequence alignment program, a pairwise sequence comparison algorithm, etc., which known in the art, may be appropriately used.

Further, the corresponding amino acid residue in another polypeptide may be identified by multiple sequence alignment. Examples of the multiple sequence alignment program known in the art comprise programs such as MUSCLE (multiple sequence comparison by log-expectation; version 3.5 or later; Edgar, 2004, Nucleic Acids Research 32: 1792-1797), MAFFT (6.857 or later; Katoh and Kuma, 2002, Nucleic Acids Research 30: 3059-3066; Katoh et al., 2005, Nucleic Acids Research 33: 511-518; Katoh and Toh, 2007, Bioinformatics 23: 372-374; Katoh et al., 2009, Methods in Molecular Biology 537: 39-64; Katoh and Toh, 2010, Bioinformatics 26: 1899-1900), and EMBOSS EMMA employing ClustalW (1.83 or later; Thompson et al., 1994, Nucleic Acids Research 22 : 4673-4680), etc., and their respective default parameters may be used, but are not limited thereto.

In addition, when polypeptides diverged from the mature polypeptide of SEQ ID NO: 1 fail to detect their relationship by traditional sequence-based comparisons, other pairwise sequence comparison algorithms may be used (Lindahl and Elofsson, 2000, J. Mol. Biol. 295: 613-615). Higher sensitivity in sequence-based searching may be attained using search programs that utilize probabilistic representations of polypeptide families (profiles) to search databases. For example, the PSI BLAST program generates profiles through an iterative database search process and is capable of detecting remote homologs (Atschul et al., 1997, Nucleic Acids Res. 25: 3389-3402). Even greater sensitivity may be achieved if the family or superfamily for the polypeptide has one or more representatives in the protein structure databases. Programs such as GenTHREADER (Jones, 1999, J. Mol. Biol. 287: 797-815; McGuffin and Jones, 2003, Bioinformatics 19: 874-881) utilize information from a variety of sources, such as PSI-BLAST, secondary structure prediction, structural alignment profiles, and solvation potentials, as input to a neural network that predicts the structural fold for a query sequence. Similarly, the method of Gough et al., 2000, J. Mol. Biol. 313: 903-919 may be used to align a sequence of unknown structure with the superfamily models present in the SCOP database. These alignments may be used in sequence to generate homology models for the polypeptides, and such models may be assessed for accuracy using a variety of tools developed for that purpose.

For proteins of known structure, several tools and resources are available for retrieving and generating structural alignments. For example, the SCOP superfamilies of proteins have been structurally aligned, and those alignments are accessible and downloadable. Two or more protein structures may be aligned using a variety of algorithms such as the distance alignment matrix (Holm and Sander, 1998, Proteins 33: 88-96) or combinatorial extension (CE) (Shindyalov and Bourne, 1998, Protein Engineering 11: 739-747). Implementation of these algorithms may be additionally utilized to query structure databases with a structure of interest in order to discover possible structural homologs (Holm and Park, 2000, Bioinformatics 16: 566-567).

The above methods are illustrative, and are not limited thereto.

In the present disclosure, with regard to the amino acid sequence, it is obvious that a polypeptide or protein "comprising" an amino acid sequence represented by a specific sequence number, a polypeptide or protein "consisting of" an amino acid sequence represented by a specific sequence number, or a polypeptide or protein "having" an amino acid sequence represented by a specific sequence number may also comprise a polypeptide or protein in which some amino acid(s) are deleted, modified, substituted, or added, as long as it has identical or corresponding activity to that of the polypeptide or protein consisting of the amino acid sequence of the corresponding sequence number. For example, the polypeptide or protein may also comprise a polypeptide or protein having additions or deletions of amino acid(s), which do not alter the function of the protein, inside or upstream or downstream (N-terminus or C-terminus) of the polypeptide or protein, naturally occurring mutations, silent mutations thereof, or conservative substitutions, as long as it has the identical or corresponding activity.

Further, for example, a polypeptide or protein conjugated with an N-terminal signal (or leader) sequence that is co-translationally or post-translationally involved in the protein (polypeptide) translocation, or a polypeptide or protein conjugated with other sequences or linkers so as to identify, purify, or synthesize the polypeptide or protein may also be comprised within the scope of the polypeptide or protein of the amino acid sequence represented by the specific sequence number.

As used herein, the term "conservative substitution" means substitution of one amino acid with another amino acid having similar structural and/or chemical properties. Such an amino acid substitution may generally occur based on similarity in polarity, charge, solubility, hydrophobicity, hydrophilicity, and/or amphipathic nature of the residues. For example, positively charged (basic) amino acids comprise arginine, lysine, and histidine; negatively charged (acidic) amino acids comprise glutamic acid and aspartic acid; amino acids having non-polar side chains (non-polar amino acids) comprise glycine, alanine, valine, leucine, isoleucine, methionine, phenylalanine, tryptophan, and proline; amino acids having polar or hydrophilic side chains (polar amino acids) comprise serine, threonine, cysteine, tyrosine, asparagine, and glutamine. For another example, amino acids may be classified into amino acids with electrically charged side chains (electrically charged amino acids) such as arginine, lysine, histidine, glutamic acid, and aspartic acid, and amino acids with uncharged side chains (uncharged amino acids; also called neutral amino acids) such as glycine, alanine, valine, leucine, isoleucine, methionine, phenylalanine, tryptophan, proline, serine, threonine, cysteine, tyrosine, asparagine, and glutamine. For still another example, phenylalanine, tryptophan, and tyrosine may be classified as aromatic amino acids. For still another example, valine, leucine, and isoleucine may be classified as branched amino acids. For still another example, 20 types of amino acids are classified according to size, starting from the amino acid group with relatively small volume, the amino acids may be classified into five groups; glycine, alanine, serine; cysteine, proline, threonine, aspartic acid, asparagine; valine, histidine, glutamic acid, glutamine; isoleucine, leucine, methionine, lysine, arginine; and phenylalanine, tryptophan, tyrosine. However, they are not necessarily limited thereto. Usually, conservative substitution may hardly affect or not affect activity of polypeptides or proteins.

As used herein, the term "different amino acid" is not limited as long as it is an amino acid different from the amino acid before substitution. Meanwhile, in the present disclosure, it is expressed that "a specific amino acid has been substituted", it is obvious that the amino acid is substituted with an amino acid different from the amino acid before substitution, even though it is not specifically stated that the amino acid has been substituted with a different amino acid.

In the present disclosure, the term "varying/modifying" refers to changing or altering. This may be alteration from naturally occurring. For example, a polypeptide may be altered in such a way that the polypeptide is changed from a parent sequence or reference sequence.

In the present disclosure, the modified polypeptide may be a polypeptide not existing per se in nature, i.e., a non-naturally occurring polypeptide.

As used herein, the term "modified" refers to, for example, altered from a naturally occurring form. A modified polypeptide of the present disclosure comprises a non-naturally occurring polypeptide or a naturally occurring variant. For example, the modified polypeptide of the present disclosure is a modified polypeptide not found in nature. For example, the modified polypeptide of the present disclosure may not spontaneously occur, but is not limited thereto.

When used in connection with an amino acid/base (nucleic acid) sequence, the term "modification" used herein may comprise substitution of an amino acid/nucleotide of a parent sequence at one or more sites of the parent amino acid/base (nucleotide) sequence with a different amino acid/nucleic acid, deletion of an amino acid/ nucleotide (or a series of amino acids/nucleotides) at one or more sites of the parent amino acid/base (nucleotide) sequence, addition of an amino acid/nucleotide (or a series of amino acids/nucleotides) at one or more sites of the parent amino acid/base (nucleotide) sequence, or any combination thereof. For example, the deletion may comprise truncation of an amino acid(s) of the N-terminus and/or the C-terminus or a 5' and/or 3' nucleotide(s), but is not limited thereto.

As used herein, the term "variant" or "variant polypeptide" refers to a polypeptide which is different from the parent sequence due to the presence of conservative substitution and/or modification (substitution, addition, or deletion, etc.) of one or more amino acids in the parent sequence. The functions or properties of such a variant polypeptide may be increased, unchanged, or decreased, as compared to that of the native polypeptide. For example, some variant polypeptides may comprise variant polypeptides in which one or more regions, such as an N-terminal leader sequence or transmembrane domain, have been removed. Further, for example, another variant polypeptide may comprise variants in which a region has been removed from the N- and/or C-terminus of a mature protein. The term "variant polypeptide" may be used interchangeably with terms such as modification, modified protein, mutant, mutein, divergent, variant, etc., and is not limited thereto as long as it is a term used with the meaning of variation.

Further, the variant polypeptide may comprise deletions or additions of amino acids that have minimal effect on the properties and secondary structure of the polypeptide. For example, the polypeptide may be conjugated with a polypeptide N-terminal signal (or leader) sequence that is co-translationally or post-translationally involved in the protein translocation. Further, the polypeptide may be conjugated with other sequences or linkers so as to identify, purify, or synthesize the polypeptide.

The variant polypeptide of the present disclosure may be in an isolated form.

As used herein, the term "isolated" refers to a substance present in an environment which does not occur naturally or present in a form which does not exist naturally. This comprises that a substance (sequence or nucleic acid) is at least substantially free from at least other components having the substance, e.g., sequence or nucleic acid, which is naturally associated in nature and as found in nature.

For example, the isolated sequence or nucleic acid provided in the present disclosure may be provided in a form that is substantially free of one or more contaminants.

Examples of the isolated substance may comprise i) any non-naturally occurring substance, ii) any substance from which one or more, or all naturally-occurring component associated in nature are removed (e.g., enzyme, variant, nucleic acid, protein, peptide, or cofactor), iii) any substance that has been artificially modified from a substance found in nature, or iv) a substance modified to change the amount of the substance compared to other components naturally associated therewith (e.g., by increasing the copy number of a gene encoding a particular substance; by modifying a promoter naturally associated with a gene encoding a particular substance with a promoter having stronger activity, etc.), but are not limited thereto.

The variant polypeptide of the present disclosure may comprise biologically active fragments of the variant polypeptide.

In the present discourse, in relation to an amino acid or base (nucleic acid) sequence, the term "biologically active fragments or fragments" may refer to "functional fragments". The "functional fragment" may also refer to an active fragment, and refers to a polypeptide that comprises fewer amino acids than a full-length protein but possesses at least one biological activity of the corresponding full-length protein. For example, the functional fragment of the enzyme may comprise a catalytic site of the enzyme.

The biologically active fragments of the variant polypeptide of the present disclosure may comprise a part of the full-length of the native polypeptide. For example, it may comprise at least about 20% or more, 30% or more, 40% or more, 50% or more, 60% or more, 70% or more, 75% or more, 80% or more, 85% or more, 90% or more, 95% or more, 96% or more, 97% or more, or 98% or more; and less than 100% of the amino acids of the full-length of the native polypeptide, but is not limited thereto.

Throughout the specification, the conventional one-letter and three-letter codes for naturally occurring amino acids are used. In addition, the amino acids mentioned herein are abbreviated according to the nomenclature rules of IUPAC-IUB.

| | |
|---|---|
| alanine Ala, A | arginine Arg, R |
| asparagine Asn, N | aspartic acid Asp, D |
| cysteine Cys, C | glutamic acid Glu, E |
| glutamine Gln, Q | glycine Gly, G |
| histidine His, H | isoleucine Ile, I |
| leucine Leu, L | lysine Lys, K |
| methionine Met, M | phenylalanine Phe, F |
| proline Pro, P | serine Ser, S |
| threonine Thr, T | tryptophan Trp, W |
| tyrosine Tyr, Y | valine Val, V |

Meanwhile, any amino acid may be described as Xaa, X.

Also, three-letter codes generally allowed for other amino acids, such as 2-aminoisobutyric acid (Aib), N-methylglycine (Sar), and α-methyl-glutamic acid, may be used as well as the naturally occurring amino acids.

Amino acids may generally be classified based on similarity in polarity, charge, solubility, hydrophobicity, hydrophilicity, and/or amphipathic nature of residues.

Examples of this classification may comprise positively charged (basic) amino acids such as arginine, lysine, and histidine; negatively charged (acidic) amino acids such as glutamic acid and aspartic acid; amino acids with non-polar side chains (non-polar amino acids) such as glycine, alanine, valine, leucine, isoleucine, methionine, phenylalanine, tryptophan, and proline; amino acids with polar or hydrophilic side chains (polar amino acids) such as serine, threonine, cysteine, tyrosine, asparagine, and glutamine. For another example, amino acids may be classified into amino acids with electrically charged side chains (electrically charged amino acids) such as arginine, lysine, histidine, glutamic acid, and aspartic acid, and amino acids with uncharged side chains (uncharged amino acids; also called neutral amino acids) such as glycine, alanine, valine, leucine, isoleucine, methionine, phenylalanine, tryptophan, proline, serine, threonine, cysteine, tyrosine, asparagine, and glutamine. For still another example, phenylalanine, tryptophan, and tyrosine may be classified as aromatic amino acids. For still another example, valine, leucine, and isoleucine may be classified as branched amino acids. For still another example, 20 types of amino acids are classified according to size, starting from the amino acid group with relatively small volume, the amino acids may be classified into five groups; glycine, alanine, serine; cysteine, proline, threonine, aspartic acid, asparagine; valine, histidine, glutamic acid, glutamine; isoleucine, leucine, methionine, lysine, arginine; and phenylalanine, tryptophan, tyrosine. However, they are not necessarily limited thereto.

In order to describe the variant provided in the present disclosure, the following nomenclature is used.

In the present disclosure, referring to a specific position in an amino acid sequence may comprise referring to an amino acid present or substituted at that position. Referring to an amino acid at a specific position may be described in various ways. For example, the "position 003" may be described as "position 3", "amino acid 3", "3^{rd} amino acid". Further, for example, when the amino acid at position 3 is serine (S), it may be described as "S3" or "Ser3".

Amino acid substitution may be expressed by describing the amino acid before substitution, the position, and the amino acid to be substituted in sequence. The amino acids may be expressed using the conventional one-letter and three-letter codes. For example, when alanine, an amino acid at position 6 of a specific sequence, is substituted with valine, it may be described as "A8V" or "Ala8Val".

Any amino acid at a specific position may be referred to as "X". For example, X6 refers to any amino acid at position 6. In addition, when the amino acid to be substituted is expressed as X, it means that the amino acid is substituted with an amino acid different from the amino acid present before substitution. For example, "V6X" indicates that V at position 6 is substituted with any amino acid other than V.

### Gene, Polynucleotide

As used herein, the term "gene" narrowly means a polynucleotide encoding a functional molecule, and broadly means a polynucleotide comprising the polynucleotide encoding the functional molecule, and upstream and downstream regions of the polynucleotide. In one specific embodiment, the functional molecule may be an RNA or a protein, and the gene may have a sequence (intron) inserted between respective coding regions (exons).

As used herein, the term "polynucleotide", "nucleic acid", or "nucleic acid molecule" refers to a DNA (e.g., cDNA or genomic DNA) or RNA (e.g., mRNA) strand of a certain length or longer as a polymer of nucleotides in which nucleotide monomers are connected in a long chain form by covalent bonds. In the present disclosure, "polynucleotide", "nucleic acid", and "nucleic acid molecule" may be used interchangeably with each other.

### Homology, Identity

As used herein, the term "identity" or "homology" refers to a degree of relevance between two given amino acid or nucleotide sequences, and may be expressed as a percentage. In the present disclosure, "homology" and "identity" may often be used interchangeably with each other.

The sequence homology or identity of a conserved polynucleotide or polypeptide is determined by standard alignment algorithms, and the default gap penalty established by a program used may be used together.

Whether or not any two polynucleotide or polypeptide sequences have homology, similarity, or identity may be determined using known computer algorithms such as the "FASTA" program, for example, using default parameters as in Pearson et al (1988) [Proc. Natl. Acad. Sci. USA 85]: 2444. Alternatively, it may be determined using Needleman-Wunsch algorithm (Needleman and Wunsch, 1970, J. Mol. Biol. 48: 443-453) as performed in the Needleman program of the EMBOSS package (EMBOSS: The European Molecular Biology Open Software Suite, Rice et al., 2000, Trends Genet. 16: 276-277) (version 5.0.0 or later) or by comparing sequence information using the GAP computer program such as Smith-Waterman algorithm (Smith and Waterman, Adv. Appl. Math (1981) 2:482) (comprising GCG program package (Devereux, J., et al, Nucleic Acids Research 12: 387 (1984)), BLASTP, BLASTN, FASTA (Atschul, [S.] [F.,] [ET AL, J MOLEC BIOL 215]: 403 (1990); Guide to Huge Computers, Martin J. Bishop, [ED.,] Academic Press, San Diego,1994, and [CARILLO et al.](1988) SIAM J Applied Math 48: 1073). For example, the homology, similarity, or identity may be determined using BLAST or ClustalW of the National Center for Biotechnology Information.

Further, whether or not any two polynucleotide sequences have a homology, similarity, or identity may be determined by Southern hybridization experiments under appropriate hybridization conditions, and the appropriate hybridization conditions may be determined by a method well known to those skilled in the art (e.g., J. Sambrook et al., Molecular Cloning, A Laboratory Manual; F.M. Ausubel et al., Current Protocols in Molecular Biology, John Wiley & Sons, Inc., New York), but are not limited thereto. For example, homologous or identical polynucleotide sequences may generally hybridize along the entirety of the sequence or at least about 50%, 60%, 70%, 80% or 90% of the full-length of the sequence under stringent conditions.

As used herein, the term "stringent conditions" refers to conditions under which specific hybridization between polynucleotides is allowed. Such conditions are specifically described in a literature (see Sambrook et al., supra, 9.50-9.51, 11.7-11.8). For example, the stringent conditions may comprise conditions under which polynucleotides having high homology or identity, polynucleotides having 60% or more, 70% or more, 75% or more, 80% or more, 85% or more, 90% or more, 95% or more, 96% or more, 97% or more, 98% or more, or 99% or more homology or identity are hybridized with each other, and polynucleotides having homology or identity lower than the above are not hybridized with each other, or washing conditions of the common Southern hybridization, i.e., washing once, specifically, twice or three times at a salt concentration and a temperature corresponding to 60°C, 1XSSC, 0.1% SDS, specifically 60°C, 0.1XSSC, 0.1% SDS, and more specifically 68°C, 0.1XSSC, 0.1% SDS.

The hybridization may occur between nucleotides having bases of sequences complementary to each other, but hybridized polynucleotides may comprise some mismatches between bases depending on the stringency of hybridization. The term "complementary" is used to describe the relation between nucleotide bases capable of being hybridized with each other. For example, with regard to DNA, adenosine is complementary to thymine and cytosine is complementary to guanine. Hence, the polynucleotide of the present disclosure may also comprise substantially similar nucleotide sequences as well as isolated nucleic acid fragments that are complementary to the entire sequence.

For example, a polynucleotide having homology or identity to the polynucleotide of the present disclosure may be detected by hybridization at a Tm value of 55°C. Further, the Tm value may be 60°C, 63°C, or 65°C, but is not limited thereto, and may be appropriately adjusted by those skilled in the art.

The appropriate stringency for hybridizing the polynucleotide depends on the length and degree of complementarity of the polynucleotide, and the variables are well known in the art (e.g., J. Sambrook et al., supra).

### Nucleic Acid Construct, Vector, Transformation

As used herein, the term "nucleic acid construct" means an artificially designed single- or double-stranded nucleic acid molecule comprised in a vector that may be used to integrate a target genetic material into an appropriate suitable host or host cell. For example, the nucleic acid construct may comprise a transgene delivered via a transformation vector that allows the inserted sequence to be replicated and/or expressed in the host cell. For example, the transgene may be replicated from an existing sequence or artificially synthesized.

As used herein, the term "vector" means a DNA construct for delivering a desired polynucleotide into an appropriate host or host cell.

For example, the vector may comprise a nucleotide sequence of a polynucleotide encoding a desired polypeptide operably linked to an appropriate expression regulatory region (regulatory sequence) so as to express the desired polypeptide in an appropriate host cell. The expression regulatory region may comprise a promoter capable of initiating transcription, any operator sequence for regulating the transcription, a sequence encoding a suitable mRNA ribosome-binding site, and a sequence for regulating termination of transcription and translation. Once transformed into an appropriate host cell (microorganism), the vector may replicate or function independently from the host genome, or may integrate into genome thereof to replicate or function.

Further, for example, the vector of the present disclosure may comprise a sequence for inserting the desired polynucleotide into the chromosome. Insertion of the polynucleotide into the chromosome using the vector may be performed by any method known in the art, for example, homologous recombination, but it is not limited thereto.

The vector used in the present disclosure is not particularly limited, but any vector known in the art may be used. Examples of vectors commonly used may comprise natural or recombinant plasmids, cosmids, viruses, and bacteriophages. For example, pWE15, M13, MBL3, MBL4, IXII, ASHII, APII, t10, t11, Charon4A, Charon21A, etc. may be used as a phage vector or a cosmid vector, and pDZ system, pDC system, pBR system, pUC system, pBluescript II system, pGEM system, pTZ system, pCL system, pET system, etc. may be used as a plasmid vector. For example, pDZ, pDC, pDCM2, pACYC177, pACYC184, pCL, pECCG117, pUC19, pBR322, pMW118, pCC1BAC vector, etc. may be used.

The vector may further comprise a selection marker to confirm transformation into a host cell, or furthermore, insertion into the chromosome in the host cell. The selection marker is for selecting the cells transformed with the vector, or for confirming insertion of the desired polynucleotide into the chromosome, and markers that provide selectable phenotypes, such as drug resistance, auxotrophy, resistance to cytotoxic agents, or expression of surface polypeptides, may be used. Only cells expressing the selection marker are able to survive or exhibit different phenotypes under the environment treated with the selective agent, and thus the transformed cells may be selected.

As used herein, the term "transformation" means that a desired polynucleotide or a vector comprising the same is introduced into a host cell (microorganism) to change the genetic trait of the host cell (microorganism). The transformed polynucleotide may be inserted into the chromosome of the host cell (microorganism) or may be located outside the chromosome. Further, the polynucleotide may comprise DNA or RNA. The polynucleotide may be introduced in an appropriate form depending on the purpose of introduction. For example, the polynucleotide for expressing the desired polypeptide may be introduced into a host cell (microorganism) in the form of an expression cassette, which is a gene construct comprising all elements required for self-expression. The expression cassette may usually comprise a promoter operably linked to a coding sequence of the desired polypeptide, a transcription termination signal, a ribosome binding site, and a translation termination signal. The expression cassette may be in the form of an expression vector capable of self-replicating. Further, the polynucleotide may be introduced into a host cell (microorganism) in its own form and operably linked to a sequence required for expression in the host cell (microorganism), but is not limited thereto.

As used herein, the term "operably linked" means a configuration in which a regulatory sequence is placed at an appropriate position such that the regulatory sequence directs expression of the coding sequence. Thus, the term "operably linked" comprises an attachment or linking between a regulatory region of a functional domain having a known or desired activity, such as a promoter, a terminator, a signal sequence, or an enhancer, and a target (gene or polypeptide) such that the expression, secretion, or function of the target may be regulated in accordance with the known or desired activity. For example, the term means that the polynucleotide sequence is functionally linked to a promoter sequence that initiates and mediates transcription of the polynucleotide encoding the polypeptide.

As used herein, the term "expression" comprises any process involved in the production of a polypeptide, e.g., transcription, post-transcriptional modification, translation, post-translational modification, and secretion, etc., but is not limited thereto.

As used herein, the term "expression vector" refers to a linear or circular nucleic acid molecule comprising a desired polynucleotide sequence and an operably linked regulatory sequence for expression thereof. For example, the expression vector may comprise a nucleotide sequence of a polynucleotide encoding a desired polypeptide operably linked to an appropriate expression regulatory region (expression regulatory sequence) so as to be able to express the desired polypeptide in an appropriate host cell.

As used herein, the term "regulatory sequence" refers to a polynucleotide sequence necessary for regulating the expression of a desired polynucleotide sequence. Each regulatory sequence may be a native (derived from the same origin) or foreign (derived from different genes) sequence to the coding sequence, or a mutant sequence or other artificial sequence thereof. Examples of the regulatory sequence may comprise a leader sequence, a polyadenylation sequence, a pro-peptide sequence, a promoter, a signal peptide sequence, an operator sequence, a sequence encoding a ribosome-binding domain, and a sequence regulating the termination of transcription and translation. The minimum units of the regulatory sequence may comprise a promoter, and a sequence for terminating transcription and translation.

As used herein, the term "genetic recombination" refers to a natural or artificial process whereby elements of a gene, such as DNA or RNA, are changed from their original sequence during the disassembly and reassembly process.

As used herein, the term "recombinant gene" means a gene having a new genetic configuration resulting from genetic recombination, such as chemical synthesis or genetic engineering techniques, etc. In the present disclosure, the terms "recombinant gene," "recombinant DNA," and "recombinant polynucleotide" may be used interchangeably with each other. For example, the recombinant gene may comprise an artificial combination of nucleic acid fragments, such as regulatory sequences, which are not found together in nature.

As used herein, the term "recombinant protein" means a protein resulting from genetic recombination.

### Microorganism

As used herein, the term "microorganism (or strain)" comprises all of wild-type microorganisms or prokaryotic or eukaryotic microorganisms in which a genetic modification naturally or artificially occurs, and it may be a microorganism in which a specific mechanism is weakened or increased due to insertion of a foreign gene or an activity enhancement or inactivation of an endogenous gene, etc., and it may be a microorganism comprising a genetic modification for production of a desired polypeptide, protein, or product. As used herein, the term "microorganism", "strain", "host", and "host cell" may be used interchangeably with each other.

As used herein, the term "recombinant microorganism" refers to a microorganism that has been genetically modified such that it exhibits a different genotype and/or phenotype compared to a naturally occurring microorganism (e.g., when the genetic modification affects coding of the base (nucleic acid) sequence of the microorganism), and may comprise any progeny or potential progeny of the microorganism. As used herein, the term "recombinant microorganism", "genetically modified microorganism", "recombinant host cell", "recombinant cell" and "recombinant strain" may be used interchangeably with each other. The recombinant microorganism, for example, may express a gene that is not found within the native (non-recombinant) form; may not express a gene that is expressed in the native form, or may express a native gene in a manner different from that expressed within the native form.

For example, the microorganism of the present disclosure may be a microorganism comprising any one or more of the variant polypeptide of the present disclosure, the polynucleotide of the present disclosure, and the vector comprising the polynucleotide of the present disclosure; a microorganism which is modified to express the variant polypeptide of the present disclosure or the polynucleotide of the present disclosure; a recombinant microorganism expressing the variant polypeptide of the present disclosure or the polynucleotide of the present disclosure; or a recombinant microorganism having activity of the variant polypeptide of the present disclosure, but is not limited thereto.

As used herein, the term "microorganism having an L-amino acid production ability" is a prokaryotic or eukaryotic microorganism capable of producing an L-amino acid in a living organism, and the microorganism may comprise all of a microorganism that inherently has the L-amino acid production ability, as well as a microorganism that does not inherently have the L-amino acid production ability but is endowed with the L-amino acid production ability. The L-amino acid production ability may be endowed or enhanced by expression of the variant polypeptide of the present disclosure or by species improvement.

As used herein, the term "unmodified microorganism (strain)" does not exclude microorganisms (strains) comprising mutations that may naturally occur, and may be a wild-type microorganism (strain) or natural microorganism (strain) itself or may be a microorganism (strain) before the trait is changed by genetic variation due to natural or artificial factors. As used herein, the term "unmodified microorganism (strain)" may be used interchangeably with "microorganism (strain) before being modified", "unvaried microorganism (strain)", "parent microorganism", "parent strain", "wild-type microorganism (strain)", "reference microorganism (strain)", or "standard microorganism (strain)". In the present disclosure, the unmodified microorganism may mean a microorganism (strain) in which the variant polypeptide of the present disclosure is not introduced or has not yet been introduced; or a microorganism (strain) comprising the wild-type polypeptide, but is not limited thereto. Further, in the present disclosure, the unmodified microorganism may be a microorganism comprising the polypeptide consisting of SEQ ID NO: 1 or a polynucleotide consisting of SEQ ID NO: 2, but is not limited thereto.

### Increase of protein (polypeptide) activity

As used herein, the term "increase" of a protein (polypeptide) activity means that the protein (polypeptide) activity in a host cell (microorganism) is increased as compared to the endogenous activity. The increase may be used interchangeably with terms such as activation, up-regulation, overexpression, enhancement, etc. The host cell (microorganism) may be a prokaryotic or eukaryotic microorganism.

The increase of the protein (polypeptide) activity may comprise both exhibiting the protein (polypeptide) activity that was not endogenously possessed by the host cell (microorganism) and exhibiting the improved protein (polypeptide) activity, as compared to the endogenous activity or activity before modification.

For example, "exhibiting the protein (polypeptide) activity that was not endogenously possessed" or "exhibiting the improved protein (polypeptide) activity" may be caused by "introduction of the protein (polypeptide)", but is not limited thereto.

As used herein, the term "introduction" of the protein (polypeptide) means exhibiting activity of a particular protein as a gene not originally possessed by a microorganism is expressed in the microorganism, or exhibiting enhanced, increased, or improved polypeptide activity, as compared with the endogenous activity of the corresponding protein or the activity before modification. For example, it may be caused by introduction of a gene encoding the protein (polypeptide) into the host cell (microorganism). For example, a polynucleotide encoding a particular protein (polypeptide) may be introduced into the chromosome of the host cell (microorganism) or a vector comprising the polynucleotide encoding the particular protein (polypeptide) may be introduced into the host cell (microorganism) to exhibit or improve its activity.

The "endogenous activity" means the activity of a specific protein (polypeptide) originally possessed by a host cell (microorganism) before change of the trait or an unmodified host cell (microorganism) when the trait is changed by genetic variation due to natural or artificial factors. This may be used interchangeably with "activity before modification".

The fact that the activity of a protein (polypeptide) is increased, as compared to the endogenous activity means that the activity and/or concentration (expression level) of the protein (polypeptide) of a host cell (microorganism) is improved, as compared to the activity and/or concentration (expression level) of the protein (polypeptide) originally possessed by the host cell (microorganism) before the trait is changed or an unmodified host cell (microorganism).

For example, the increase indicates that the activity of the corresponding protein (polypeptide) originally absent appears, or its activity or concentration is generally increased to about 1% or more, about 10% or more, about 25% or more, about 50% or more, about 75% or more, about 100% or more, about 150% or more, about 200% or more, about 300% or more, about 400% or more or about 500% or more, up to about 1000% or about 2000% or more, based on the activity or concentration in the host cell (microorganism) before the trait is changed or the unmodified host cell (microorganism), but is not limited thereto.

The increase of the protein (polypeptide) activity may be achieved through the introduction of a foreign protein (polypeptide) or the increase of the endogenous protein (polypeptide) activity. The increase of the protein (polypeptide) activity may be confirmed by an increase in the degree of activity and the expression level of the corresponding protein (polypeptide) or an increase in the amount of a product released from the corresponding protein (polypeptide) activity.

The increase of the protein (polypeptide) activity may be achieved by various methods well known in the art, and the method is not limited as long as the activity of the desired protein (polypeptide) may be increased as compared to that of the host cell (microorganism) before modification. Specifically, genetic engineering and/or protein engineering well known to those skilled in the art, which are routine methods of molecular biology, may be used, but the method is not limited thereto (e.g., Sitnicka et al. Functional Analysis of Genes. Advances in Cell Biology. 2010, Vol. 2. 1-16, Sambrook et al. Molecular Cloning 2012, etc.).

Specifically, the increase of the activity of the protein (polypeptide) of the present disclosure may be:
1) increase in the intracellular copy number of the polynucleotide encoding the protein (polypeptide);
2) modification of a gene expression regulatory region on a chromosome encoding the protein (polypeptide) (e.g., introduction of variations into the expression regulatory region, replacement with a sequence having stronger activity, or insertion of a sequence having stronger activity;
3) modification of a nucleotide sequence encoding an initiation codon or 5'-UTR region of the gene transcript encoding the protein (polypeptide);
4) modification of the amino acid sequence of the protein (polypeptide) to increase the activity of the protein (polypeptide);
5) modification of the polynucleotide sequence encoding the protein (polypeptide) to increase the activity of the protein (polypeptide) (e.g., modification of the polynucleotide sequence of the protein (polypeptide)-encoding gene to encode the protein (polypeptide) that has been modified to increase the activity of the protein (polypeptide));
6) introduction of a foreign protein (polypeptide) exhibiting the activity of the protein (polypeptide) or a foreign polynucleotide encoding the same;
7) codon optimization of the polynucleotide encoding the protein (polypeptide);
8) analysis of the tertiary structure of the protein (polypeptide) to select and to modify or chemically modify the exposed site;
9) control of intracellular localization of the protein (polypeptide); or
10) a combination of two or more selected from 1) to 9), but is not particularly limited thereto.

For example,
1) the increase in the intracellular copy number of the polynucleotide encoding the protein (polypeptide) may be achieved by the introduction, into a host cell (microorganism), of a vector comprising the polynucleotide encoding the protein (polypeptide), which is operably linked to an appropriate regulatory sequence. Alternatively, the increase may be achieved by introducing one copy or two or more copies of the polynucleotide encoding the protein (polypeptide), which is operably linked to an appropriate regulatory sequence, into the chromosome of a host cell (microorganism). The introduction into the chromosome may be performed by the introduction, into the host cell (microorganism), of a vector capable of inserting the polynucleotide into the chromosome of the host cell (microorganism), but is not limited thereto. The vector is as described above. The regulatory sequence may be a native (derived from the same origin) or foreign (derived from different genes) sequence to the coding polynucleotide sequence, or a mutant sequence or other artificial sequence thereof, and may induce expression of the polynucleotide in the host cell (microorganism).
2) The replacement of a gene expression regulatory region (or expression regulatory sequence) on the chromosome encoding the protein (polypeptide) with a sequence with strong activity may be, for example, introduction of a mutation on the sequence through deletion, insertion, substitution, or a combination thereof, or replacement with a sequence having stronger activity, in order to further increase the activity of the expression regulatory region. The expression regulatory region may comprise, but is not particularly limited to, a promoter, an operator sequence, a sequence encoding a ribosome binding site, a sequence controlling the termination of transcription and translation, etc. For example, it may be the replacement of the original promoter with a stronger promoter, but is not limited thereto.
   Examples of the known stronger promoter may comprise cj1 to cj7 promoters (US Patent No. 7662943 B2), lac promoter, trp promoter, trc promoter, tac promoter, lambda phage PR promoter, PL promoter, tet promoter, gapA promoter, SPL7 promoter, SPL13(sm3) promoter (US Patent No. 10584338 B2), O2 promoter(US Patent No. 10273491 B2), tkt promoter, yccA promoter, etc., but are not limited thereto.
3) The modification of a nucleotide sequence encoding an initiation codon or 5'-UTR region of the gene encoding the protein (polypeptide) may be, for example, replacing with another initiation codon having a higher protein (polypeptide) expression rate than the endogenous initiation codon, or modifying for encoding ribosome binding site (RBS) sequence having a higher protein (polypeptide) expression rate than the endogenous RBS sequence, but is not limited thereto.
4) and 5) The modification of the amino acid sequence or polynucleotide sequence of the protein (polypeptide) may be performed by introducing a mutation into the amino acid sequence of the protein (polypeptide) or the polynucleotide sequence encoding the protein (polypeptide) by deletion, insertion, substitution, or a combination thereof to increase the activity of the protein (polypeptide), or by replacing with an amino acid sequence or a polynucleotide sequence which is modified to increase the activity, but is not limited thereto. The replacement may be performed by, for example, inserting the polynucleotide into the chromosome by homologous recombination, but is not limited thereto.
6) The introduction of a foreign polynucleotide exhibiting the activity of the protein (polypeptide) may be introduction, into a host cell (microorganism), of a foreign polynucleotide encoding a protein (polypeptide) exhibiting activity identical or similar to that of the protein (polypeptide). There is no limitation on its origin or sequence as long as the foreign polynucleotide exhibits activity identical or similar to that of the protein (polypeptide). The method used in the introduction may be performed by appropriately selecting a known transformation method by those skilled in the art. As the introduced polynucleotide is expressed in the host cell, the protein (polypeptide) may be produced and the activity thereof may be increased.
7) The codon optimization of the polynucleotide encoding the protein (polypeptide) may be codon optimization of an endogenous polynucleotide so as to enhance transcription or translation in a host cell (microorganism), or codon optimization of a foreign polynucleotide so as to perform optimized transcription and translation in a host cell (microorganism).
8) The analysis of the tertiary structure of the protein (polypeptide) to select and to modify or chemically modify the exposed site may be, for example, to determine a template protein candidate according to the degree of similarity of the sequence by comparing the sequence information of a protein (polypeptide) to be analyzed with a database storing the sequence information of known proteins, to confirm the structure based on this, and to modify or chemically modify the exposed site to be modified or chemically modified.
9) The control of cellular localization of the protein (polypeptide) may be targeting the protein (polypeptide) to a specific organelle within cells or to a specific intracellular space. For example, it may be targeting to the periplasm or cytoplasm through the addition or removal of a leader sequence that functions in targeting the protein (polypeptide), but is not limited thereto.

Such an increase of the activity of the protein (polypeptide) may be an increase of the activity or concentration of the corresponding protein (polypeptide), based on the activity or concentration of the protein (polypeptide) expressed in a wild-type or a host cell (microorganism) before modification, or an increase in the amount of a product resulting from the activity of the corresponding protein (polypeptide), but is not limited thereto.

The modification of a part or the entirety of the polynucleotide in the host cell (microorganism) of the present disclosure may be induced by: (a) homologous recombination using a vector for chromosomal insertion or genome editing using engineered nuclease (e.g., CRISPR-Cas9), and/or (b) the treatment with light, such as ultraviolet light and radiation, and/or chemicals, but is not limited thereto.

### Culture

As used herein, the term "culturing" refers to growing a microorganism in appropriately adjusted environment conditions. The culture procedure may be performed according to appropriate media or culture conditions known in the art. Such culturing process may be easily adjusted and used by a person skilled in the art according to the selected microorganism. Specifically, the culturing may be in a batch type, a continuous type, and/or a fed-batch type, but is not limited thereto.

As used herein, the "medium" refers to a mixture comprising, as main ingredients, nutrient materials required for culturing a microorganism, wherein the medium supplies nutrient materials comprising water essential for survival and growth, growth factors, etc. Specifically, as for the media and other culture conditions used for culturing the microorganism of the present disclosure, any medium that is commonly used for culturing microorganisms may be used without particular limitation. For example, the microorganism of the present disclosure may be cultured under aerobic conditions in a common medium comprising appropriate carbon sources, nitrogen sources, phosphorus sources, inorganic compounds, amino acids, and/or vitamins, etc., while controlling the temperature, pH, etc. For example, a culture medium for microorganisms of the genus *Corynebacterium* may be found in the literature ["Manual of Methods for General Bacteriology" by the American Society for Bacteriology (Washington D.C., USA, 1981)].

In the present disclosure, the carbon source may comprise carbohydrates, such as glucose, saccharose, lactose, fructose, sucrose, maltose, etc.; sugar alcohols, such as mannitol, sorbitol, etc.; organic acids, such as pyruvic acid, lactic acid, citric acid, etc.; and amino acids, such as glutamic acid, methionine, lysine, etc. In addition, natural organic nutrient sources may be used, such as starch hydrolysates, molasses, blackstrap molasses, rice bran, cassava, bagasse, and corn steep liquor, and specifically, carbohydrates such as glucose and sterile pretreated molasses (i.e., molasses converted to reduced sugars) may be used, and appropriate amounts of various other carbon sources may be used without limitation. These carbon sources may be used alone or in a combination of two or more thereof, but are not limited thereto.

As for the nitrogen sources, inorganic nitrogen sources, such as ammonia, ammonium sulfate, ammonium chloride, ammonium acetate, ammonium phosphate, ammonium carbonate, ammonium nitrate, etc.; amino acids, such as glutamic acid, methionine, glutamine, etc.; and organic nitrogen sources, such as peptone, NZ-amine, meat extracts, yeast extracts, malt extracts, corn steep liquor, casein hydrolysates, fishes or decomposition products thereof, defatted soybean cake or degradation products thereof, etc. may be used. These nitrogen sources may be used alone or in a combination of two or more thereof, but are not limited thereto.

The phosphorus sources may comprise potassium phosphate monobasic, potassium phosphate dibasic, and sodium-comprising salts corresponding thereto. As for inorganic compounds, sodium chloride, calcium chloride, iron chloride, magnesium sulfate, iron sulfate, manganese sulfate, calcium carbonate, etc. may be used, and in addition, amino acids, vitamins, and/or suitable precursors may be comprised. These constituent ingredients or precursors may be added to the medium in a batch or continuous manner. However, the present disclosure is not limited thereto.

Further, pH of the medium may be adjusted by adding compounds, such as ammonium hydroxide, potassium hydroxide, ammonia, phosphoric acid, sulfuric acid, etc., to the medium in an appropriate manner during culturing of the microorganism of the present disclosure. In addition, an anti-foaming agent, such as a fatty acid polyglycol ester, may be used to suppress foam formation during culturing. In addition, oxygen or oxygen-comprising gas may be injected into the medium to maintain the aerobic state of the medium, or no gas may be injected or nitrogen, hydrogen or carbon dioxide gas may be injected to maintain the anaerobic or nonaerobic state, but is not limited thereto.

In the culturing of the present disclosure, the culture temperature may be maintained at 20°C to 45°C, specifically 25°C to 40°C, and the culturing may be performed for about 10 hours to 160 hours, but is not limited thereto.

As used herein, the term "culture" refers to a culture liquid obtained by culturing a specific microorganism in a culture medium, a concentrated culture liquid, a dry product of the culture liquid, a culture filtrate, a concentrated culture filtrate, or a dry product of the culture filtrate, and the culture liquid refers to those comprising the specific microorganism, and the culture filtrate refers to those substantially not comprising the specific microorganism (which means that the specific microorganism to be separated by filtration, etc. are substantially excluded, which does not mean that the microorganism is completely excluded from the filtrate.). The formulation of the culture is not limited, but it may be, for example, a liquid, an emulsion, or a solid.

As used herein, the term "fermentation" refers to a process in which microorganisms break down organic materials using their own enzymes, excluding putrefaction. Fermentation and putrefaction proceed by similar processes, but when useful substances are produced as a result of decomposition, it is called fermentation, and when bad smells or harmful substances are produced, it is called putrefaction.

In the present disclosure, the method of obtaining the fermentation product from the microorganism is not particularly limited, and the fermentation product may be obtained according to a method commonly used in the art or similar art.

As used herein, the term "fermentation product" comprises not only a fermented material itself, but also all types of materials comprising the fermentation product generated from the microorganism, such as a material comprising the fermented microorganism, a culture generated from the fermented microorganism, a fermentation product of the culture, a concentrated fermentation product, a dry product of the fermentation product, a filtrate of the fermentation product, a filtrate of the concentrated fermentation product, or a dry product of the filtrate of the fermentation product, an extract of the fermentation product, or a dilution of the fermentation product, etc.

### Detailed description of the present disclosure

Hereinafter, specific embodiments of the present disclosure will be described in more detail as follows.

An aspect of the present disclosure provides a variant polypeptide of ribose phosphate diphosphokinase, in which an amino acid corresponding to position 239 in an amino acid sequence of SEQ ID NO: 1 is substituted with a different amino acid.

As used herein, the term "ribose phosphate diphosphokinase (PRSA)" refers to an enzyme that converts ribose 5-phosphate into phosphoribosyl pyrophosphate. The ribose phosphate diphosphokinase of the present disclosure may be used interchangeably with PRSA(Phosphoribosyl pyrophosphate synthetase).

Specifically, the ribose phosphate diphosphokinase protein of the present disclosure may be a protein having the activity of ribose phosphate diphosphokinase encoded by *prsA* gene, but is not limited to the type, as long as it has the activity corresponding to ribose phosphate diphosphokinase. The ribose phosphate diphosphokinase protein encoded by *prsA* gene is known in the art, and amino acid and polynucleotide sequences of the ribose phosphate diphosphokinase protein may be obtained from known databases, for example, NCBI's Genbank, etc., but are not limited thereto.

For example, the ribose phosphate diphosphokinase protein that is a target of mutation introduction of the present disclosure may comprise the amino acid sequence of SEQ ID NO: 1 or an amino acid sequence having 60% or more homology or identity thereto, but is not limited thereto, as long as it has the ribose phosphate diphosphokinase activity. Specifically, it is apparent that any protein comprising a sequence with deletion, modification, substitution, or addition in part of the amino acid sequence of SEQ ID NO: 1 may also be comprised in the ribose phosphate diphosphokinase protein, as long as it is a protein exhibiting efficacy corresponding to that of the ribose phosphate diphosphokinase. Further, any protein may be comprised in the ribose phosphate diphosphokinase protein as long as it has an amino acid sequence having at least 60%, 70%, 80%, 85%, 90%, 95%, 96%, 97%, 98%, or 99% or more homology or identity to the amino acid sequence of SEQ ID NO: 1, comprises the amino acid sequence, consists of the amino acid sequence, or essentially consists of the amino acid sequence while exhibiting the efficacy corresponding to that of the ribose phosphate diphosphokinase.

Further, a sequence of a polynucleotide encoding the ribose phosphate diphosphokinase protein having the amino acid sequence of SEQ ID NO: 1 or an amino acid sequence having 60% or more homology or identity thereto may be obtained, for example, based on codon information known in the art. For example, the ribose phosphate diphosphokinase protein may be encoded by a polynucleotide having or comprising a sequence of SEQ ID NO: 2 or a nucleotide sequence having 60% or more, 70% or more, 75% or more, 80% or more, 85% or more, 90% or more, 95% or more, 96% or more, 97% or more, 98% or more, or 99% or more homology or identity to the sequence of SEQ ID NO: 2, or consisting of or essentially consisting of the nucleotide sequence, but is not limited thereto. Further, the nucleotide sequence of SEQ ID NO: 2 may be obtained from known databases, for example, NCBI's Genbank, etc., but is not limited thereto.

In the present disclosure, the polynucleotide (gene) comprising the nucleotide sequence of SEQ ID NO: 2 may be used interchangeably with a polynucleotide (gene) having the nucleotide sequence of SEQ ID NO: 2, a polynucleotide (gene) consisting of the nucleotide sequence of SEQ ID NO: 2, or *prsA.*

The variant polypeptide of ribose phosphate diphosphokinase of the present disclosure may be a variant polypeptide, in which an amino acid corresponding to position 239 in the amino acid sequence of SEQ ID NO: 1 is substituted with a different amino acid.

The variant polypeptide of ribose phosphate diphosphokinase of the present disclosure may comprise those in which the amino acid corresponding to position 239 from the N-terminus of the amino acid sequence of SEQ ID NO: 1 is substituted with a different amino acid, in the amino acid sequence of SEQ ID NO: 1 or an amino acid sequence having at least 60%, 70%, 75%, 80%, 85%, 90%, 95%, 96%, 97%, 98%, 99%, 99.5%, 99.7%, or 99.9% or more homology or identity to the amino acid sequence of SEQ ID NO: 1. Further, it is apparent that any variant having an amino acid sequence with deletion, substitution, conservative substitution, or addition in part of the sequence may also fall within the scope of the present disclosure, as long as it is an amino acid sequence comprising the above amino acid substitution, having the above homology or identity, and exhibiting efficacy corresponding to that of the variant of the present disclosure.

The "different amino acid" is not limited as long as it is an amino acid different from the amino acid before substitution. Meanwhile, in the present disclosure, when it is expressed that "a specific amino acid has been substituted", it is obvious that the amino acid is substituted with an amino acid different from the amino acid before substitution, even though it is not stated that the amino acid has been substituted with a different amino acid.

In the present disclosure, when it is expressed that "the amino acid corresponding to position 239 of the amino acid sequence of SEQ ID NO: 1 has been substituted with a different amino acid", it may mean that the amino acid is substituted with glutamate, phenylalanine, glycine, arginine, aspartate, cysteine, asparagine, glutamine, histidine, proline, serine, tyrosine, isoleucine, lysine, tryptophan, valine, methionine, threonine, or leucine, excluding alanine, but is not limited thereto.

In any one embodiment of the above-described embodiments, the variant polypeptide of ribose phosphate diphosphokinase provided in the present disclosure may be a variant polypeptide in which the amino acid corresponding to position 239 from the N-terminus of the amino acid sequence of SEQ ID NO: 1 is substituted with any one amino acid selected from glycine, alanine, valine, leucine, isoleucine, methionine, phenylalanine, tryptophan, proline, serine, threonine, cysteine, tyrosine, asparagine, and glutamine, which are amino acids having uncharged side chains.

In any one embodiment of the above-described embodiments, the variant polypeptide of ribose phosphate diphosphokinase of the present disclosure may be a variant polypeptide in which the amino acid corresponding to position 239 of the amino acid sequence of SEQ ID NO: 1 is substituted with valine, proline, asparagine, methionine, glycine, or tyrosine.

For example, the variant polypeptide of ribose phosphate diphosphokinase of the present disclosure may comprise an amino acid sequence having at least 60% or more, 70% or more, 75% or more, 80% or more, 85% or more, 90% or more, 95% or more, 96% or more, 97% or more, or 98% or more; and less than 100% homology or identity to SEQ ID NO: 1 while the amino acid corresponding to position 239 of the amino acid sequence represented by SEQ ID NO: 1, valine, proline, asparagine, methionine, glycine, or tyrosine is fixed. Further, it is apparent that any variant polypeptide of ribose phosphate diphosphokinase having an amino acid sequence with deletion, modification, substitution, conservative substitution, or addition in part of the sequence may also fall within the scope of the present disclosure, as long as it is an amino acid sequence having the above homology or identity and exhibiting efficacy corresponding to that of the variant polypeptide of the ribose phosphate diphosphokinase of the present disclosure.

**In** another specific embodiment, the variant polypeptide may consist of any one amino acid sequence selected from SEQ **ID** NOS: 15 to 20.

Specifically, the variant polypeptide of the present disclosure may have or comprise any one selected from SEQ **ID** NOS: 15 to 20 or an amino acid sequence having at least 60%, 70%, 80%, 85%, 90%, 95%, 96%, 97%, 98%, or 99% or more homology or identity to any one sequence selected from SEQ **ID** NOS: 15 to 20, or may consist of the amino acid sequence, or may essentially consist of the amino acid sequence.

Another aspect of the present disclosure provides a polynucleotide encoding the variant polypeptide of ribose phosphate diphosphokinase.

The polynucleotide encoding the variant polypeptide of ribose phosphate diphosphokinase of the present disclosure may comprise any polynucleotide sequence without limitation as long as it is a polynucleotide sequence encoding the variant polypeptide of the present disclosure. The polynucleotide may be prepared, based on codon information known in the art, but is not limited thereto. For example, the polynucleotide may comprise, consist of, or essentially consist of any one nucleotide sequence selected from SEQ **ID** NOS: 47 to 52.

**In** the polynucleotide of the present disclosure, various modifications may be made in the coding region as long as the amino acid sequence of the variant polypeptide of the present disclosure is not changed due to codon degeneracy or in consideration of codons preferred in organisms that are intended to express the variant polypeptide of the present disclosure. Therefore, it is obvious that, due to codon degeneracy, a polynucleotide to be translated into a polypeptide consisting of the amino acid sequence of the variant polypeptide of the present disclosure or a polypeptide having homology or identity thereto may also be comprised. For example, the polynucleotide of the present disclosure may be any one nucleotide sequence selected from SEQ ID NOS: 47 to 52 or a degenerated sequence thereof.

For another example, the polynucleotide of the present disclosure may have or comprise any one nucleotide sequence selected from SEQ ID NOS: 47 to 52 or a nucleotide sequence having 60% or more, 70% or more, 75% or more, 80% or more, 85% or more, 90% or more, 95% or more, 96% or more, 97% or more, 98% or more, or 99% or more homology or identity thereto, may consist of or essentially consist of the nucleotide sequence, but is not limited thereto. For example, the polynucleotide of the present disclosure may comprise a nucleotide sequence having a fixation of the codon encoding the amino acid corresponding to position 239 of any one of SEQ ID NOS: 15 to 20 with any one of codons encoding valine, proline, asparagine, methionine, glycine, or tyrosine.

Further, the polynucleotide of the present disclosure may comprise a probe that may be prepared from a known gene sequence, for example, may comprise any sequence without limitation as long as it is a sequence that is able to hybridize with a complementary sequence to the entirety or a part of the polynucleotide sequence of the present disclosure under stringent conditions and encodes the variant polypeptide of the present disclosure. The stringent conditions are as explained above.

Still another aspect of the present disclosure provides a vector comprising the polynucleotide. The vector may be an expression vector for expressing the polynucleotide in a microorganism, but is not limited thereto.

Still another aspect of the present disclosure provides a microorganism comprising the variant polypeptide of ribose phosphate diphosphokinase or the polynucleotide encoding the variant polypeptide.

In one specific embodiment, the microorganism of the present disclosure may be a microorganism having an L-amino acid production ability.

In the present disclosure, the L-amino acid may be specifically any one or more selected from the group consisting of L-tryptophan and L-histidine.

The microorganism of the present disclosure comprises all of a microorganism that comprises the variant polypeptide sequence of ribose phosphate diphosphokinase of the present disclosure due to a mutation of the gene encoding the polypeptide of ribose phosphate diphosphokinase of the present disclosure on the chromosome; and/or a microorganism that comprises the variant polypeptide of ribose phosphate diphosphokinase of the present disclosure by introducing the polynucleotide encoding the variant polypeptide of ribose phosphate diphosphokinase of the present disclosure or the vector comprising the same, but is not limited thereto.

With respect to the object of the present disclosure, the microorganism of the present disclosure may comprise any microorganism comprising the variant polypeptide of ribose phosphate diphosphokinase of the present disclosure and capable of producing a desired L-amino acid. For example, the microorganism of the present disclosure may be a microorganism that is transformed with the vector comprising the polynucleotide encoding the variant polypeptide of ribose phosphate diphosphokinase of the present disclosure to express the variant polypeptide of the present disclosure, thereby having the increased L-amino acid production ability, but is not limited thereto. For another example, the microorganism of the present disclosure may be a microorganism having the increased L-amino acid production ability by expressing the variant polypeptide of the present disclosure due to introduction of the polynucleotide encoding the variant polypeptide of ribose phosphate diphosphokinase of the present disclosure into a native wild-type microorganism or a microorganism producing an L-amino acid, but is not limited thereto. The microorganism having the increased L-amino acid production ability may be a genetically engineered microorganism or a recombinant microorganism, and the microorganism having the increased L-amino acid production ability may be a microorganism in which the L-amino acid production ability is increased, as compared to a native wild-type microorganism or unmodified microorganism (e.g., a microorganism expressing the wild-type polypeptide or the polypeptide comprising the parent sequence; or a microorganism not expressing the variant polypeptide of ribose phosphate diphosphokinase of the present disclosure), but is not limited thereto.

For example, the microorganism having the L-amino acid production ability of the present disclosure may comprise any microorganism which is transformed with the vector to express the variant polypeptide of ribose phosphate diphosphokinase of the present disclosure and to produce an L-amino acid.

The microorganism of the present disclosure may comprise any microorganism capable of expressing the variant polypeptide of ribose phosphate diphosphokinase of the present disclosure by various known methods, in addition to the introduction of the polynucleotide or vector.

For example, the microorganism having the increased L-amino acid production ability of the present disclosure may be a microorganism in which the L-amino acid production ability is increased, as compared to the parent microorganism (parent strain) before modification or the unmodified microorganism (e.g., a microorganism expressing the wild-type polypeptide, the polypeptide comprising the parent sequence, or the polypeptide of SEQ ID NO: 1; or a microorganism not expressing the variant polypeptide of ribose phosphate diphosphokinase of the present disclosure), but is not limited thereto. For example, the parent microorganism (parent strain) before modification or the unmodified microorganism for comparing whether or not the L-amino acid production ability is increased may be a CM05-9157 (Korean Patent No. 10-2278000), CA14-0809 (KCCM12489P, Korean Patent No. 10-2019-0046934), CM05-9157β or CA14-0809βprsA(B.su) strain, but is not limited thereto.

For example, the microorganism having the increased L-amino acid production ability may have an increased L-amino acid production ability of about 1% or more, specifically, about 1% or more, about 2.5% or more, about 5% or more, about 6% or more, about 7% or more, about 8% or more, about 9% or more, about 10% or more, about 10.5% or more, about 11% or more, about 11.5%or more, about 12% or more, about 12.5% or more, about 13% or more, about 13.5% or more, about 14% or more, about 14.5% or more, about 15% or more or about 15.5% or more (the upper limit is not particularly limited, but may be, for example, about 200% or less, about 150% or less, about 100% or less, about 50% or less, about 45% or less, about 40% or less, about 35% or less, about 30% or less, or about 25% or less), as compared to the parent microorganism (parent strain) before modification or unmodified microorganism, but the increased amount is not limited thereto as long as the production ability has an increased amount of a + value, as compared to the production ability of the parent microorganism (parent strain) before modification or the unmodified microorganism. In another example, the recombinant microorganism having the increased L-amino acid production ability may have an L-amino acid production ability of about 1.1 times or more, about 1.12 times or more, about 1.13 times or more, about 1.14 times or more, or about 1.15 times or more (the upper limit is not particularly limited, but may be, for example, about 10 times or less, about 5 times or less, about 3 times or less, about 2 times or less, about 1.5 times or less, about 1.45 times or less, about 1.4 times or less, about 1.35 times or less, about 1.3 times or less, or about 1.25 times or less), as compared to the parent microorganism (parent strain) before modification or the unmodified microorganism, but is not limited thereto.

For example, the microorganism having the L-amino acid production ability may be either prokaryotic cell or eukaryotic cell, specifically, prokaryotic cell. The prokaryotic cell may comprise, for example, microorganisms of *Escherichia* sp., *Erwinia* sp., *Serratia* sp., *Providencia* sp., *Corynebacterium* sp., *Pseudomonas* sp., *Leptospira* sp., *Salmonella* sp., *Brevibacteria* sp., *Hypomononas* sp., *Chromobacterium* sp., and *Norcardia* sp., but is not limited thereto. Specifically, the prokaryotic cell may be a microorganism of *Escherichia* sp. or *Corynebacterium* sp. More specifically, the prokaryotic cell may be a microorganism of *Corynebacterium* sp.

The microorganism of *Corynebacterium* sp. may be *Corynebacterium glutamicum, Corynebacterium crudilactis, Corynebacterium deserti, Corynebacterium efficiens, Corynebacterium callunae, Corynebacterium stationis, Corynebacterium singulare, Corynebacterium halotolerans, Corynebacterium striatum, Corynebacterium ammoniagenes, Corynebacterium pollutisoli, Corynebacterium imitans, Corynebacterium testudinoris,* or *Corynebacterium flavescens.* Specifically, the microorganism of the present disclosure may be *Corynebacterium glutamicum,* but is not limited thereto.

Meanwhile, it has already been known that microorganisms of the *Corynebacterium* sp. are able to produce an L-amino acid, but the genes involved in the production mechanisms or the principle of mechanisms have not been fully elucidated. Therefore, the microorganism of the *Corynebacterium* sp. having the L-amino acid production ability of the present disclosure may comprise all of a natural wild-type microorganism itself, a microorganism of the *Corynebacterium* sp. having the improved L-amino acid production ability by strengthening or weakening the activity of genes related to the L-amino acid production mechanism, or a microorganism of the *Corynebacterium* sp. having the improved L-amino acid production ability by introducing or strengthening activity of a foreign gene.

Still another aspect of the present disclosure provides a method of producing an L-amino acid, the method comprising the step of culturing, in a medium, the microorganism comprising the variant polypeptide of ribose phosphate diphosphokinase of the present disclosure, in which the amino acid corresponding to position 239 in the amino acid sequence of SEQ ID NO: 1 is substituted with a different amino acid, or the polynucleotide encoding the variant polypeptide.

With regard to the method of the present disclosure, the variant polypeptide of ribose phosphate diphosphokinase, polynucleotide, microorganism, and L-amino acid, etc. are as described in other aspects.

With regard to the method of the present disclosure, the culturing of the microorganism may be performed using any culture conditions and culture methods known in the art. Such culturing process may be easily adjusted and used by a person skilled in the art according to the selected microorganism.

The L-amino acid produced by the culturing of the present disclosure may be released into the medium or may remain in cells.

In one specific embodiment, the method of producing an L-amino acid of the present disclosure may further comprise the steps of preparing the microorganism of the present disclosure, preparing a medium for culturing the microorganism, or a combination thereof (regardless of the order, in any order), for example, before the culturing step.

The method of producing an L-amino acid of the present disclosure may further comprise the step of recovering the target material, specifically, L-amino acid from the cultured microorganism, a culture of the microorganism, a fermentation product of the microorganism, or the culture medium. The recovering step may be further comprised after the culturing step.

The recovering may be collecting a desired L-amino acid by using an appropriate method known in the art according to the method of culturing the microorganism of the present disclosure, for example, a batch, continuous, or fed-batch type culture. For example, centrifugation, filtration, treatment with a crystallized protein precipitating agent (salting-out), extraction, sonication, ultrafiltration, dialysis, various types of chromatography, such as molecular sieve chromatography (gel filtration), adsorption chromatography, ion exchange chromatography, and affinity chromatography, etc., HPLC, and a combination of these methods may be used, and the target material, specifically, L-amino acid may be recovered from the medium or microorganism by using an appropriate method known in the art.

In addition, the method of producing an L-amino acid of the present disclosure may further comprise a purification step. The purification may be performed by using an appropriate method known in the art. In an exemplary embodiment, when the method of producing an L-amino acid of the present disclosure comprises both the recovering step and the purification step, the recovering step and the purification step may be performed continuously or discontinuously regardless of the order, or may be performed simultaneously or integrated into one step, but is not limited thereto.

Still another aspect of the present disclosure provides a composition for producing an L-amino acid, the composition comprising the variant polypeptide of ribose phosphate diphosphokinase of the present disclosure; the polynucleotide encoding the variant polypeptide; the microorganism comprising the variant polypeptide or the polynucleotide encoding the variant polypeptide; the culture of the microorganism; a combination of two or more thereof.

With regard to the composition of the present disclosure, the variant polypeptide of ribose phosphate diphosphokinase, polynucleotide, microorganism, culture and L-amino acid, etc. are as described in other aspects.

The composition of the present disclosure may further comprise any appropriate excipient that is usually used in the composition for producing L-amino acids, and such excipients may comprise, for example, a preserving agent, a wetting agent, a dispersing agent, a suspending agent, a buffering agent, a stabilizing agent, an isotonic agent, etc., but are not limited thereto.

In one specific embodiment, each component present in the composition of the present disclosure may be comprised in a microbiologically effective amount, or in an amount that may be appropriately present in the composition for production.

Still another aspect of the present disclosure provides use of the variant polypeptide of ribose phosphate diphosphokinase of the present disclosure; the microorganism comprising the variant polypeptide or the polynucleotide encoding the variant polypeptide; or the culture of the microorganism in producing an L-amino acid.

With regard to the use of the present disclosure, the variant polypeptide of ribose phosphate diphosphokinase, microorganism, L-amino acid, etc. are as described in other aspects.

### [Mode for Carrying Out the Invention]

Hereinafter, the present disclosure will be described in more detail by way of exemplary embodiments. However, the following exemplary embodiments are only preferred embodiments for illustrating the present disclosure, and thus are not intended to limit the scope of the present disclosure thereto. Meanwhile, technical matters not described in the present specification may be sufficiently understood and easily implemented by those skilled in the technical field of the present disclosure or similar technical fields.

### Example 1. Preparation of L-Tryptophan-Producing Microorganism Introduced with Bacillus Subtilis-Derived Ribose Phosphate Diphosphokinase

### Example 1-1. Construction of vector for gene insertion

In order to insert a gene into the chromosome of *Corynebacterium,* a plasmid pDCM2 (Korean Patent No. 10-2278000) was used as a parent vector, and to enhance the activity of ribose phosphate diphosphokinase, a plasmid for further inserting prsA gene was constructed using Pcj7 promoter (Korean Patent No. 10-0620092).

In detail, chromosomal DNA of the wild-type *Corynebacterium glutamicum* ATCC13869 was used as a template, and the upstream and downstream of the region where homologous recombination on the chromosome occurs were amplified using a primer pair of SEQ ID NO: 3 and SEQ ID NO: 4 and a primer pair of SEQ ID NO: 5 and SEQ ID NO: 6, respectively, and respective gene fragments were obtained. The sequences of the used primers are as shown in Table 1 below.

**[Table 1]**

| SEQ ID NO. | Primer name | Sequence (5'→3') |
|---|---|---|
| 3 | HL1 F | aacgacggccagtgaattcTCGTTGGCCATTACCTCAT |
| 4 | HL1 R | ctgtttAGTACTaaaccggaagggccTTATCTGCCACACCC ACAA |
| 5 | HR1 F | cggtttAGTACTaaacaggaagagccTAAAAACGGAAGAG CCACT |
| 6 | HR1 R | ttgcatgcctgcaggtcgacGGAAGACTATCCAAGGTGG |

To obtain the above fragments, PCR was performed. Solg^{™} Pfu-X DNA polymerase was used as a polymerase, and PCR amplification was performed under conditions of denaturation at 95°C for 4 minutes, followed by 27 cycles of denaturation at 95°C, annealing at 55°C, and polymerization at 72°C, and then polymerization at 72°C for 5 minutes.

The upstream fragment and downstream fragment of the region where homologous recombination on the chromosome occurs, which were obtained through the above process, were cloned into a chromosomal transformation vector pDCM2 digested with EcoRI and Sall restriction enzymes using the Gibson assembly method (DG Gibson et al., NATURE METHODS, VOL.6 NO.5, MAY 2009, NEBuilder HiFi DNA Assembly Master Mix) to obtain a recombinant plasmid, which was named pDCM2-ΔTn.

### Example 1-2. Construction of vector for introducing Bacillus subtilis-derived ribose phosphate diphosphokinase

First, to obtain the Pcj7 promoter, PCR was performed using p117-cj7-gfp (US 7662943 B2) as a template and primers of SEQ ID NO: 7 and SEQ ID NO: 8 in Table 2 below. Solg^{™} Pfu-X DNA polymerase (SolGent co.) was used as a polymerase, and the PCR amplification conditions were denaturation at 95°C for 2 minutes, followed by 27 cycles of denaturation at 95°C, annealing at 55°C, and polymerization at 72°C, and then polymerization at 72°C for 5 minutes.

Ribose phosphate diphosphokinase derived from *Bacillus subtilis* has an amino acid sequence represented by SEQ ID NO: 1. A gene encoding ribose phosphate diphosphokinase and its surrounding nucleotide sequence (accession number NC_000964.3, SEQ ID NO: 2) were obtained from the National Institutes of Health (NIH) GenBank. Based on the obtained nucleotide sequence, primers for inserting the *Bacillus subtilis-derived* gene into the genomic DNA of *Corynebacterium glutamicum* were synthesized.

The ribose phosphate diphosphokinase gene of *Bacillus subtilis* was synthesized using the gene synthesis service of Bionics Co., Ltd., and PCR was performed using primers of SEQ ID NO: 9 and SEQ ID NO: 10 in Table 2 below. Solg^{™} Pfu-X DNA polymerase was used as a polymerase, and PCR amplification conditions were denaturation at 95°C for 2 minutes, 27 cycles of denaturation at 95°C for 20 seconds, annealing at 55°C, polymerization at 72°C for 1 minute, and then polymerization reaction at 72°C for 5 minutes.

**[Table 2]**

| SEQ ID NO. | Primer name | Sequence (5'→3') |
|---|---|---|
| 7 | P_Pcj7_ref-F | taaggcccttccggtttagtAGAAACATCCCAGCGCT ACT |
| 8 | P_Pcj7(B. su)_ref-R | tctccgtattgattagacatGAGTGTTTCCTTTCGTT GGG |
| 9 | G_prs(B. su)-F | cccaacgaaaggaaacactcATGTCTAATCAATACG GAGA |
| 10 | G_prs(B. su)-R | ttaggctcttcctgtttagtTTAGCTGAACAGATAGCT GA |

Subsequently, the Pcj7 promoter region thus amplified, the *Bacillus subtilis-*derived gene fragment, and a chromosomal transformation vector pDCM2-△Tn digested with the Scal restriction enzyme were cloned using the Gibson assembly method (DG Gibson et al., NATURE METHODS, VOL.6 NO.5, MAY 2009, NEBuilder HiFi DNA Assembly Master Mix) to obtain a recombinant plasmid, which was named pDCM2-β. The cloning was performed by mixing the Gibson assembly reagent and each gene fragment in the calculated numbers of moles and then storing the mixture at 50°C for 1 hour.

### Example 1-3. Preparation of L-tryptophan-producing microorganism introduced with Bacillus subtilis-derived ribose phosphate diphosphokinase

The pDCM2-β vector constructed in Example 1-2 was transformed into an L-tryptophan producing strain CM05-9157 (Korean Patent No. 10-2278000) by electroporation (Appl. Microbiol.Biotechnol. (1999) 52:541-545), and then a secondary crossover process was performed to obtain a strain in which one copy of the Pcj7-prsA(B.su) gene was inserted between transposon genes on the chromosome. Thereafter, the gene manipulation was examined through PCR using primers of SEQ ID NO: 13 and SEQ ID NO: 14 in Table 3 below, which are able to amplify external regions of the homologous recombinant upstream and downstream regions where the corresponding gene was inserted, and genome sequencing.

**[Table 3]**

| | SEQ ID NO. | Name | Sequence (5'→ 3') |
|---|---|---|---|
| | 13 | Confirm-Pcj7-prs-F | GTTCGGCTTGGATTATGG |
| | 14 | Confirm-Pcj7-prs-R | CATCAACAACAGCCTTCA |

The strain obtained through the method described above was named CM05-9157β.

### Example 2. Assessment of L-Tryptophan Production Ability of L-Tryptophan-Producing Microorganism Introduced with Bacillus Subtilis-Derived Ribose Phosphate Diphosphokinase

In order to examine the L-tryptophan production ability of the CM05-9157β strain prepared in Example 1-3 and the parent strain CM05-9157, the strains were cultured using the following method and medium compositions.

First, each strain was inoculated into a 250 ml corner-baffled flask comprising 25 ml of a seed medium, and cultured at 30°C for 20 hours under shaking at 200 rpm. Then, 1 ml of the seed culture was inoculated into a 250 ml corner-baffled flask comprising 25 ml of a production medium, and cultured at 30°C for 24 hours under shaking at 200 rpm. After the culture was completed, the production amount of L-tryptophan was measured by HPLC.

### <Seed medium (pH 7.0)>

20 g of glucose, 10 g of peptone, 5 g of yeast extract, 1.5 g of urea, 4 g of KH₂PO₄, 8 g of K₂HPO₄, 0.5 g of MgSO₄7H₂O, 100 µg of biotin, 1000 µg of thiamine HCl, 2000 µg of calcium-pantothenic acid, 2000 µg of nicotinamide (based on 1 liter of distilled water)

### <Production medium (pH 7.0)>

30 g of glucose, 15 g of (NH₄)₂SO₄, 1.2 g of MgSO₄·7H₂O, 1 g of KH₂PO₄, 5 g of yeast extract, 900 µg of biotin, 4500 µg of thiamine HCl, 4500 µg of calcium-pantothenic acid, 30 g of CaCO₃ (based on 1 liter of distilled water)

As a result of the experiment, the L-tryptophan production amount of each strain was as in Table 4 below.

**[Table 4]**

| **Strain** | **OD562** | **Production amount of tryptophan (g/L)** | **Yield of tryptophan (*100 g/g, %)** |
|---|---|---|---|
| CM05-9157 | 56.3 | 1.88 | 6.37 |
| CM05-9157β | 53.1 | 2.29 | 7.74 |

The CM05-9157β strain, into which the ribose phosphate diphosphokinase gene derived from *Bacillus subtilis* was introduced, produced a final 2.29 g/L of L-tryptophan in the flask culture, which was a fermentation yield improvement of about 21%, as compared to the control strain, CM05-9157. This suggests that the L-tryptophan production ability of *Corynebacterium glutamicum* strain may be significantly increased by introducing the ribose phosphate diphosphokinase gene derived from *Bacillus subtilis.*

### Example 3. Preparation of L-Tryptophan-Producing Microorganism Introduced with Variant having substitution of amino acid at position 239 of Bacillus Subtilis-Derived Wild-Type Ribose Phosphate Diphosphokinase

To further increase the activity of ribose phosphate diphosphokinase derived from *Bacillus subtilis,* a variant was constructed, in which alanine, an amino acid at position 239, (hereinafter, represented by alanine at position 239, or 239^{th}) was substituted with another amino acid. For mutation into an amino acid other than alanine, a site-directed mutagenesis method was used using pDCM2-β of Example 1 as a template. The site-directed mutagenesis method was performed according to the PCR composition and PCR cycle in Tables 5 and 6 below.

**[Table 5]**

| **PCR composition for site-directed mutagenesis** | **Unit (ul)** |
|---|---|
| 10X pfu-X Buffer | 5 |
| 10mM dNTP Mix | 1 |
| pfu-X Polymerase | 1 |
| Mutagenic forward primer(5pmol) | 2 |
| Mutagenic reverse primer(5pmol) | 2 |
| pDCM2-β(template DNA, 200ng/ul) | 1 |
| dH2O | 38 |
| Total | 50 |

**[Table 6]**

| **Cycle** | **Temperature** | **Time** |
|---|---|---|
| 1 | 95°C | 1 min |
| | 95°C | 50 sec |
| 18 | 60°C | 50 sec |
| | 68°C | 8 min |
| 1 | 68°C | 7 min |

In order to substitute alanine, which is the amino acid at position 239 of the amino acid sequence of prsA (B.su), with different amino acids, valine (V) (SEQ ID NO: 15), proline (P) (SEQ ID NO: 16), asparagine (N) (SEQ ID NO: 17), methionine (M) (SEQ ID NO: 18), glycine (G) (SEQ ID NO: 19), tyrosine (Y) (SEQ ID NO: 20), respectively, each mutagenic primer set listed in Table 7 below was used to prepare a PCR mixture as in Table 5, and PCR was performed with cycles in Table 6. After PCR was completed, 1 µl of Dpnl restriction enzyme was added, and treated at 37°C for 1 hour. 3 µl of Dpnl-treated DNA was transformed into DH5a competent cells to obtain each variant pDCM2-β plasmid, and substitution with each mutation as shown in Table 7 was identified by sequencing.

**[Table 7]**

| **Variant prsA(B.su) plasmid** | **SEQ ID NO.** | **Sequence (5'-3')** |
|---|---|---|
| pDCM2-βA239V | 21 | cttgctgctaat**gtg**ctcgttgaaaacggagcgaa |
| | 22 | ctccgttttcaacgag**cac**attagcagcaagtqta |
| pDCM2-βA239P | 23 | cttgctgctaat**cca**ctcgttgaaaahggagcgaa |
| | 24 | ctccgttttcaacgag**tgg**attagcagcaagtgta |
| pDCM2-βA239N | 25 | cttgctgctaat**aac**ctcgttgaaaacqgagcgaa |
| | 26 | ctccgttttcaacgag**gtt**attagcagcaagtgta |
| pDCM2-βA239M | 27 | cttgctgctaat**atg**ctcgttgaaaacggagcgaa |
| | 28 | ctccgttttcaacgag**cat**attagcagcaagtgta |
| pDCM2-βA239G | 29 | cttgctgctaat**ggc**ctcgttgaaaacggagcgaa |
| | 30 | ctccgttttcaacgag**gcc**attagcagcaagtgta |
| pDCM2-βA239Y | 31 | cttgctgctaat**tac**ctcgttgaaaacggagcgaa |
| | 32 | ctccgttttcaacgag**gta**attagcagcaagtgta |

The prepared pDCM2-βA239V, pDCM2-βA239P, pDCM2-βA239N, pDCM2-βA239M, pDCM2-βA239G, and pDCM2-βA239Y vectors, as shown in Table 7, were transformed into the L-tryptophan-producing strain CM05-9157 prepared in Example 1-3 by electroporation, respectively, and then through a secondary crossover process, 6 types of strains were obtained, in which the variant prsA (B.su) gene was inserted on the chromosome. The gene manipulation was examined through PCR using primers of SEQ ID NO: 33 and SEQ ID NO: 34, which are able to amplify external regions of the homologous recombinant upstream and downstream regions where the corresponding gene was inserted, and genome sequencing.

The transformed strains thus obtained were named CM05-9157βprsA(B.su) A239V), CM05-9157βprsA(B.su) A239P), CM05-9157βprsA(B.su) A239N), CM05-9157βprsA(B.su) A239M), CM05-9157βprsA(B.su) A239G), CM05-9157βprsA(B.su) A239Y), respectively.

### Example 4. Assessment of L-Tryptophan Production Ability of L-Tryptophan-Producing Microorganism Introduced with Variant having substitution of amino acid at position 239 of Bacillus Subtilis-Derived Wild-Type Ribose Phosphate Diphosphokinase

In order to compare the L-tryptophan production amounts of the 6 types of strains prepared in Example 3 and CM05-9157β, they were cultured using the same method as Example 2. After the culture was completed, the production amounts of L-tryptophan were measured using HPLC.

As a result of the experiment, as shown in Table 8 below, it was confirmed that the L-tryptophan production amount of CM05-0157β strain into which the prsA(B.su) A239V mutation was introduced was 8.98 g/L, which was about 15.6% improvement in the yield, as compared to the control CM05-9157β strain into which the wild-type prsA(B.su) protein was introduced. It was also confirmed that the yield of CM05-9157βprsA(B.su) A239P) strain was improved by about 14.3%, the yield of CM05-9157βprsA(B.su) A239N) strain was improved by about 13.9%, the yield of CM05-9157βprsA(B.su) A239M) strain was improved by about 12.6%, the yield of CM05-9157βprsA(B.su) A239G) strain was improved by about 11.7%, and the yield of CM05-9157βprsA(B.su) A239Y) strain was improved by about 10.4%.

**[Table 8]**

| **Strain** | **OD562** | **Production amount of tryptophan (g/L)** | **Yield of tryptophan (*100 g/g, %)** |
|---|---|---|---|
| CM05-9157β | 52.9 | 2.30 | 7.79 |
| CM05-9157β | 53.4 | 2.66 | 8.98 |
| CM05-9157β | 53.0 | 2.63 | 8.89 |
| CM05-9157β | 53.1 | 2.62 | 8.86 |
| CM05-9157β | 52.1 | 2.59 | 8.77 |
| CM05-9157β | 52.7 | 2.57 | 8.70 |
| CM05-9157β | 52.0 | 2.54 | 8.58 |

The above results indicate that the L-tryptophan production ability of *Corynebacterium glutamicum* strain may be further increased by introducing the variant of the amino acid at position 239 of ribose phosphate diphosphokinase derived from *Bacillus subtilis.*

### Example 5. Preparation of L-Histidine-Producing Microorganism Introduced with Bacillus Subtilis-Derived Ribose Phosphate Diphosphokinase

### Example 1-1. Construction of vector for gene insertion

In order to insert a gene into the chromosome of *Corynebacterium,* a plasmid pDCM2 (Korean Patent No. 10-2278000) was used as a parent vector, and to enhance the activity of ribose phosphate diphosphokinase, a plasmid for further inserting prsA gene was constructed using Pm4ddh promoter (Korean Patent No. 10-0987281).

In detail, chromosomal DNA of the wild-type *Corynebacterium glutamicum* ATCC13032 was used as a template, and the upstream and downstream of the region where homologous recombination on the chromosome occurs were amplified using a primer pair of SEQ ID NO: 35 and SEQ ID NO: 36 and a primer pair of SEQ ID NO: 37 and SEQ ID NO: 38, respectively, and respective gene fragments were obtained. The sequences of the used primers are as shown in Table 9 below.

**[Table 9]**

| SEQ ID NO. | Primer name | Sequence (5'→3') |
|---|---|---|
| 35 | HL-F2 | GAATTCGAGCTCGGTACCCGACACATCCGCACCG GAC |
| 36 | HL-R2 | GACTTTTCTTCTTACGCTGCTGAGTACTGCTTGCTT AGAGTCCTG |
| 37 | HR-F2 | ATCAGGACTCTAAGCAAGCAGTACTCAGCAGCGTA AGAAGAAAAG |
| 38 | HR-R2 | TGCAGGTCGACTCTAGAGGATCCCCTTGATGACTC TTAGGGCTAG |

To obtain the above fragments, PCR was performed. Solg^{™} Pfu-X DNA polymerase was used as a polymerase, and PCR amplification was performed under conditions of denaturation at 95°C for 4 minutes, followed by 27 cycles of denaturation at 95°C, annealing at 55°C, and polymerization at 72°C, and then polymerization at 72°C for 5 minutes.

The upstream fragment and downstream fragment of the region where homologous recombination on the chromosome occurs, which were obtained through the above process, were cloned into a chromosomal transformation vector pDCM2 digested with Smal restriction enzyme using the Gibson assembly method (DG Gibson et al., NATURE METHODS, VOL.6 NO.5, MAY 2009, NEBuilder HiFi DNA Assembly Master Mix) to obtain a recombinant plasmid, which was named pDCM2-β.

### Example 5-2. Construction of vector for introducing Bacillus subtilis-derived ribose phosphate diphosphokinase

First, to obtain the ddhP1 promoter, PCR was performed using pDZ-ddhP1 (Korean Patent No. 10-0987281) as a template and primers of SEQ ID NO: 41 and SEQ ID NO: 42 in Table 10 below. Solg^{™} Pfu-X DNA polymerase (SolGent co.) was used as a polymerase, and the PCR amplification conditions were denaturation at 95°C for 2 minutes, followed by 27 cycles of denaturation at 95°C, annealing at 55°C, and polymerization at 72°C, and then polymerization at 72°C for 5 minutes.

Ribose phosphate diphosphokinase derived from *Bacillus subtilis* has an amino acid sequence represented by SEQ ID NO: 1. Information about a gene encoding ribose phosphate diphosphokinase and its surrounding nucleotide sequence (accession number NC_000964.3, SEQ ID NO: 2) was obtained from the National Institutes of Health (NIH) GenBank. Based on the obtained nucleotide sequence, primers for inserting the *Bacillus subtilis-derived* gene into the genomic DNA of *Corynebacterium glutamicum* were synthesized.

To amplify the gene derived from *Bacillus subtilis,* the ribose phosphate diphosphokinase gene of *Bacillus subtilis* strain was synthesized using the gene synthesis service of Bionics Co., Ltd., and PCR was performed using primers of SEQ ID NO: 39 and SEQ ID NO: 40 in Table 10 below. Solg^{™} Pfu-X DNA polymerase was used as a polymerase, and PCR amplification conditions were denaturation at 95°C for 2 minutes, 27 cycles of denaturation at 95°C for 20 seconds, annealing at 55°C, polymerization at 72°C for 1 minute, and then polymerization reaction at 72°C for 5 minutes.

**[Table 10]**

| SEQ ID NO. | Primer name | Sequence (5'→3') |
|---|---|---|
| 39 | G_prs(B. su)-F2 | CACAATTTTGGAGGATTACAAGAACATGTCTAATCAATAC GGAGATAAGA |
| 40 | G_prs(B. su)-R2 | ATTGACTTTTCTTCTTACGCTGCTGAGTTTAGCTGAACAG ATAGCTGACT |
| 41 | P_ddhP1-F | GAATCAGGACTCTAAGCAAGCAGTGCCGTGCGTGGCGA GT |
| 42 | P_ddhP1-R | TCTTATCTCCGTATTGATTAGACATGTTCTTGTAATCCTCC AAAATTGTG |

Subsequently, the Pcj7 promoter region thus amplified, the *Bacillus subtilis-*derived gene fragment, and a chromosomal transformation vector pDCM2-ΔTn2 digested with Scal restriction enzyme were cloned using the Gibson assembly method (DG Gibson et al., NATURE METHODS, VOL.6 NO.5, MAY 2009, NEBuilder HiFi DNA Assembly Master Mix) to obtain a recombinant plasmid, which was named pDCM2-β. The cloning was performed by mixing the Gibson assembly reagent and each gene fragment in the calculated numbers of moles and then storing the mixture at 50°C for 1 hour.

### Example 5-3. Preparation of L-histidine-producing microorganism introduced with Bacillus subtilis-derived ribose phosphate diphosphokinase

The pDCM2-β vector constructed in Example 5-2 was transformed into an L-histidine producing strain CA14-0809 (Korean Patent No. 10-2019-0065984) by electroporation (Appl. Microbiol.Biotechnol. (1999) 52:541-545), and then a secondary crossover process was performed to obtain a strain in which one copy of ddhP1_prsA(B.su) gene was inserted between transposon genes on the chromosome. The gene manipulation was examined through PCR using primers of SEQ ID NO: 43 and SEQ ID NO: 44 in Table 11 below, which are able to amplify external regions of the homologous recombinant upstream and downstream regions where the corresponding gene was inserted, and genome sequencing.

**[Table 11]**

| | SEQ ID NO. | Name | Sequence (5'→ 3') |
|---|---|---|---|
| | 43 | Confirm-ddhP1-prs-F | ACATCCTTCTTATTCAGCTCAG |
| | 44 | Confirm-ddhP1-prs-R | TGGTTTTAGTCCGATCGAGG |

The strain thus obtained was named CA14-0809β.

### Example 6. Assessment of L-Histidine Production Ability of L-Histidine-Producing Microorganism Introduced with Bacillus Subtilis-Derived Ribose Phosphate Diphosphokinase

In order to examine the L-tryptophan production ability of the CA14-0809β strain, into which the *Bacillus subtilis-derived* ribose phosphate diphosphokinase was introduced, prepared in Example 5-3, a histidine-producing strain CA14-0809 which is a parent strain was used as a control strain, and culturing was performed by the following method.

First, each strain was inoculated into a 250 ml corner-baffled flask comprising 25 ml of a seed medium, and cultured at 30°C for 20 hours under shaking at 200 rpm. Then, 1 ml of the seed culture was inoculated into a 250 ml corner-baffled flask comprising 25 ml of a production medium, and cultured at 30°C for 24 hours under shaking at 200 rpm. The compositions of the media used in this Example are as follows. After the culture was completed, the production amount of L-histidine was measured by HPLC.

### <Seed medium (pH 7.0)>

5% glucose, 1% bactopeptone, 0.25% sodium chloride, 1% yeast extract, 0.4% urea, pH 7.2

### <Production medium (pH 7.0)>

5% glucose, 2% ammonium sulfate, 0.1% monobasic potassium phosphate, 0.05% magnesium sulfate heptahydrate, 2.0% corn steep liquor (CSL), 200 µg/L biotin, 30 g/L calcium carbonate, pH 7.2

As a result of the experiment, the L-histidine production amount of each strain was as in Table 12 below.

**[Table 12]**

| **Strain** | **OD562** | **Production amount of histidine (g/L)** | **Yield of histidine (*100 g/g, %)** |
|---|---|---|---|
| CA14-0809 | 85.3 | 5.1 | 10.2 |
| CA14-0809β | 84.1 | 5.7 | 11.4 |

The CA14-0809β strain, into which the ribose phosphate diphosphokinase gene derived from *Bacillus subtilis* was introduced, produced a final 5.7 g/L of L-histidine in the flask culture, which was a fermentation yield improvement of about 12%, as compared to the control strain, CA14-0809. This suggests that the L-histidine production ability of *Corynebacterium glutamicum* strain may be significantly increased by introducing the ribose phosphate diphosphokinase gene derived from *Bacillus subtilis.*

### Example 7. Preparation of L-Histidine-Producing Microorganism Introduced with Variant having substitution of amino acid at position 239 of Bacillus Subtilis-Derived Wild-Type Ribose Phosphate Diphosphokinase

To further increase the activity of ribose phosphate diphosphokinase derived from *Bacillus subtilis,* it was attempted to increase L-histidine production by introducing the A239V variant, which most significantly increased L-tryptophan production in Example 4.

For mutation of alanine into valine, pDCM2-β used in Example 5-2 was used as a template, and site-directed mutagenesis was employed. The site-directed mutagenesis method was performed according to the PCR composition and PCR cycle in Tables 13 and 14 below.

**[Table 13]**

| **PCR composition for site-directed mutagenesis** | **Unit (ul)** |
|---|---|
| 10X pfu-X Buffer | 5 |
| 10mM dNTP Mix | 1 |
| pfu-X Polymerase | 1 |
| Mutagenic forward primer(5pmol) | 2 |
| Mutagenic reverse primer(5pmol) | 2 |
| pDCM2- pDCM2-β(template DNA, 200ng/ul) | 1 |
| dH2O | 38 |
| Total | 50 |

**[Table 14]**

| **Cycle** | **Temperature** | **Time** |
|---|---|---|
| 1 | 95°C | 1 min |
| 18 | 95°C | 50 sec |
| | 60°C | 50 sec |
| | 68°C | 8 min |
| 1 | 68°C | 7 min |

In order to substitute alanine, which is the amino acid at position 239 of the amino acid sequence of prsA(B.su), with a different amino acid, valine (V) (SEQ ID NO: 15), each mutagenic primer set listed in Table 15 below was used to prepare a PCR mixture as in Table 13, and PCR was performed with cycles in Table 14. After PCR was completed, 1 µl of Dpnl restriction enzyme was added, and treated at 37°C for 1 hour. 3 µl of Dpnl-treated DNA was transformed into DH5a competent cells to obtain a variant pDCM2-β plasmid, and substitution with each mutation as shown in Table 15 was identified by sequencing in the same manner as Example 4.

**[Table 15]**

| **Variant prsA(B.su) plasmid** | **SEQ ID NO.** | **Sequence (5'-3')** |
|---|---|---|
| pDCM2-βA239V | 45 | cttgctgctaat**gtg**ctcgttgaaaacggagcgaa |
| | 46 | ctccgttttcaacgag**cac**attagcagcaagtgta |

The pDCM2-βA239V vector, as prepared in Table 15, was transformed into the L-histidine-producing strain CA14-0809 (KCCM12489P, Korean Patent No. 10-2019-0046934) by electroporation, and then through a secondary crossover process, 1 type of strain was obtained, in which the variant prsA(B.su) gene was inserted on the chromosome. The gene manipulation was examined through PCR using primers of SEQ ID NO: 43 and SEQ ID NO: 44 of Table 11, which are able to amplify external regions of the homologous recombinant upstream and downstream regions where the corresponding gene was inserted, and genome sequencing. The transformed strain thus obtained was named CA14-0809βprsA(B.su)A239V.

### Example 8. Assessment of L-Histidine Production Ability of L-Histidine-Producing Microorganism Introduced with Variant having substitution of amino acid at position 239 of Bacillus Subtilis-Derived Wild-Type Ribose Phosphate Diphosphokinase

In order to compare the histidine production amounts of CA14-0809βprsA(B.su)A239V strain prepared in Example 7 and CA14-0809βprsA(B.su) strain, they were cultured using the same method as Example 6. After the culture was completed, the production amounts of L-histidine were measured using HPLC.

As a result of the experiment, as shown in Table 16 below, it was confirmed that the L-histidine production amount of CA14-0809βprsA(B.su)A239V strain into which the prsA(B.su) A239V mutation was introduced was 6.2 g/L, which was about 12.7% improvement in the yield, as compared to the control CA14-0809βprsA(B.su) strain into which the wild-type prsA(B.su) protein was introduced.

**[Table 16]**

| **Strain** | **OD562** | **Production amount of histidine (g/L)** | **Yield of histidine (*100 g/g, %)** |
|---|---|---|---|
| CA14-0809βprsA(B.su) | 85.6 | 5.5 | 11.0 |
| CA14-0809βprsA(B.su)A239V | 84.2 | 6.2 | 12.4 |

The above results indicate that the L-histidine production ability of *Corynebacterium glutamicum* strain may be further increased by introducing the variant of the amino acid at position 239 of ribose phosphate diphosphokinase derived from *Bacillus subtilis.*

Based on the above description, it will be understood by those skilled in the art that the present disclosure may be implemented in a different specific form without changing the technical spirit or essential characteristics thereof. In this regard, it should be understood that the above embodiment is not limitative, but illustrative in all aspects. The scope of the disclosure is defined by the appended claims rather than by the description preceding them, and therefore all changes and modifications that fall within metes and bounds of the claims, or equivalents of such metes and bounds are therefore intended to be embraced by the claims.

## Claims

1. A variant polypeptide of ribose phosphate diphosphokinase, wherein an amino acid corresponding to position 239 in an amino acid sequence of SEQ ID NO: 1 is substituted with a different amino acid.

2. The variant polypeptide of claim 1, wherein the amino acid corresponding to position 239 in the amino acid sequence of SEQ ID NO: 1 is substituted with valine, proline, asparagine, methionine, glycine, or tyrosine.

3. The variant polypeptide of claim 1, wherein the variant polypeptide consists of any one amino acid sequence selected from SEQ ID NOS: 15 to 20.

4. A polynucleotide encoding the variant polypeptide of ribose phosphate diphosphokinase of any one of claims 1 to 3.

5. A microorganism comprising the variant polypeptide of ribose phosphate diphosphokinase of any one of claims 1 to 3, or a polynucleotide encoding the variant polypeptide.

6. The microorganism of claim 5, wherein the microorganism has an increased L-amino acid production ability, as compared to an unmodified microorganism.

7. The microorganism of claim 5, wherein the microorganism is a microorganism of the *Corynebacterium* sp.

8. The microorganism of claim 7, wherein the microorganism of the *Corynebacterium* sp. is *Corynebacterium glutamicum.*

9. The microorganism of claim 6, wherein the L-amino acid is any one or more selected from the group consisting of L-tryptophan and L-histidine.

10. A method of producing an L-amino acid, the method comprising the step of culturing the microorganism of claim 5 in a medium.

11. The method of claim 10, further comprising the step of recovering a target material from the cultured microorganism, a culture of the microorganism, a fermentation product of the microorganism, or the culture medium.

12. A composition for producing an L-amino acid, the composition comprising the variant polypeptide of ribose phosphate diphosphokinase of any one of claims 1 to 3; a polynucleotide encoding the variant polypeptide; a microorganism comprising the variant polypeptide or the polynucleotide encoding the variant polypeptide; a culture of the microorganism; a combination of two or more thereof.

13. Use of the variant polypeptide of ribose phosphate diphosphokinase of any one of claims 1 to 3; a polynucleotide encoding the variant polypeptide; a microorganism comprising the variant polypeptide or the polynucleotide encoding the variant polypeptide; or a culture of the microorganism in producing an L-amino acid.
